# EUROPEAN PATENT SPECIFICATION

(11) **EP 2 023 954 B1**
(45) Date of publication and mention of the grant of the patent: **17.07.2013**
(21) Application number: 07809100.6
(22) Date of filing: 18.05.2007
(51) Int. Cl.: A61K 39/21

(54) **IMMUNOLOGICAL COMPOSITION**
IMMUNOLOGISCHE ZUSAMMENSETZUNG
COMPOSITION IMMUNOLOGIQUE

(30) Priority: 19.05.2006 US 801853 P
(43) Date of publication of application: 18.02.2009
(73) Proprietor: Sanofi Pasteur, Inc., Swiftwater, PA 18370 (US)
(72) Inventor: TARTAGLIA, James, Aurora, ON (CA); PANTALEO, Guiseppe, 1011 Lausanne (CH); HARARI, Alexandri, 1011 Lausanne (CH)
(74) Representative: Cole, William Gwyn
(86) International application number: PCT/US2007/011924
(87) International publication number: WO 2007/136763

(56) References cited:
- DATABASE BIOSIS [Online] BIOSCIENCES INFORMATION SERVICE, PHILADELPHIA, PA, US; 1 June 2004 (2004-06-01), CHIKHLIKAR PRIYA ET AL: "Inverted terminal repeat sequences of adeno-associated virus enhance the antibody and CD8+ responses to a HIV-1 p55Gag/LAMP DNA vaccine chimera" XP002498298 Database accession no. PREV200400325965 & VIROLOGY, vol. 323, no. 2, 1 June 2004 (2004-06-01), pages 220-232, ISSN: 0042-6822
- KUMAR SANJEEV ET AL: "Development of a candidate DNA/MVA HIV-1 subtype C vaccine for India" VACCINE, vol. 24, no. 14, March 2006 (2006-03), pages 2585-2593, XP002498295 ISSN: 0264-410X
- DATABASE BIOSIS [Online] BIOSCIENCES INFORMATION SERVICE, PHILADELPHIA, PA, US; May 2006 (2006-05), GOONETILLEKE NILU ET AL: "Induction of multifunctional human immunodeficiency virus type 1 (HIV-1)-specific T cells capable of proliferation in healthy subjects by using a prime-boost regimen of DNA- and modified vaccinia virus Ankara-vectored vaccines expressing HIV-1 gag coupled to CD8(+) T-cell epitopes" XP002498299 Database accession no. PREV200600433377 & JOURNAL OF VIROLOGY, vol. 80, no. 10, May 2006 (2006-05), pages 4717-4728, ISSN: 0022-538X
- DATABASE BIOSIS [Online] BIOSCIENCES INFORMATION SERVICE, PHILADELPHIA, PA, US; January 2006 (2006-01), CEBERE INESE ET AL: "Phase I clinical trial safety of DNA- and modified virus Ankara-vectored human immunodeficiency virus type 1 (HIV-1) vaccines administered alone and in a prime-boost regime to healthy HIV-1-uninfected volunteers" XP002498300 Database accession no. PREV200600253680 & VACCINE, vol. 24, no. 4, January 2006 (2006-01), pages 417-425, ISSN: 0264-410X
- MWAU MATILU ET AL: "A human immunodeficiency virus 1 (HIV-1) clade A vaccine in clinical trials: stimulation of HIV-specific T-cell responses by DNA and recombinant modified vaccinia virus Ankara (MVA) vaccines in humans" JOURNAL OF GENERAL VIROLOGY, vol. 85, no. Part 4, April 2004 (2004-04), pages 911-919, XP002498296 ISSN: 0022-1317
- DATABASE MEDLINE [Online] US NATIONAL LIBRARY OF MEDICINE (NLM), BETHESDA, MD, US; 1991, NAYLOR P H ET AL: "Preclinical and clinical studies on immunogenicity and safety of the HIV-1 p17-based synthetic peptide AIDS vaccine--HGP-30-KLH." XP002498301 Database accession no. NLM1823903 & INTERNATIONAL JOURNAL OF IMMUNOPHARMACOLOGY 1991, vol. 13 Suppl 1, 1991, pages 117-127, ISSN: 0192-0561
- DATABASE BIOSIS [Online] BIOSCIENCES INFORMATION SERVICE, PHILADELPHIA, PA, US; 22 May 1999 (1999-05-22), SANDSTROM ERIC ET AL: "Therapeutic immunisation with recombinant gp160 in HIV-1 infection: A randomised double-blind placebo-controlled trial" XP002498302 Database accession no. PREV199900318851 & LANCET (NORTH AMERICAN EDITION), vol. 353, no. 9166, 22 May 1999 (1999-05-22), pages 1735-1742, ISSN: 0099-5355
- DATABASE MEDLINE [Online] US NATIONAL LIBRARY OF MEDICINE (NLM), BETHESDA, MD, US; October 2004 (2004-10), CAPUTO ANTONELLA ET AL: "Recent advances in the development of HIV-1 Tat-based vaccines." XP002498303 Database accession no. NLM15544457 & CURRENT HIV RESEARCH OCT 2004, vol. 2, no. 4, October 2004 (2004-10), pages 357-376, ISSN: 1570-162X
- DATABASE MEDLINE [Online] US NATIONAL LIBRARY OF MEDICINE (NLM), BETHESDA, MD, US; 15 December 2001 (2001-12-15), HEL Z ET AL: "Potentiation of simian immunodeficiency virus (SIV)-specific CD4(+) and CD8(+) T cell responses by a DNA-SIV and NYVAC-SIV prime/boost regimen." XP002498304 Database accession no. NLM11739541 & JOURNAL OF IMMUNOLOGY (BALTIMORE, MD. : 1950) 15 DEC 2001, vol. 167, no. 12, 15 December 2001 (2001-12-15), pages 7180-7191, ISSN: 0022-1767
- DATABASE BIOSIS [Online] BIOSCIENCES INFORMATION SERVICE, PHILADELPHIA, PA, US; January 2008 (2008-01), HARARI ALEXANDRE ET AL: "An HIV-1 clade C DNA prime, NYVAC boost vaccine regimen induces reliable, polyfunctional, and long-lasting T cell responses" XP002498305 Database accession no. PREV200800151089 & JOURNAL OF EXPERIMENTAL MEDICINE, vol. 205, no. 1, January 2008 (2008-01), pages 63-77, ISSN: 0022-1007
- MCCORMACK SHEENA ET AL: "EV02: a Phase I trial to compare the safety and immunogenicity of HIV DNA-C prime-NYVAC-C boost to NYVAC-C alone." VACCINE 13 JUN 2008, vol. 26, no. 25, 13 June 2008 (2008-06-13), pages 3162-3174, XP002498297 ISSN: 0264-410X

## Description

### Field of the Invention

The disclosure relates to immunological compositions for vaccinating human beings against infection by the Human Immunodeficiency Virus (HIV).

### Background of the Invention

Globally, by the end of 2001 40 million people were estimated to be infected with HIV (UNAIDS 2001). AIDS killed 2.3 million African people in 2001 and is now the fourth commonest cause of death worldwide. Over 90% of HIV infections occur in developing countries, with the majority of infections found in sub-Saharan Africa (28.1 million) and Asia and the Pacific (7.1 million). Because of the high cost of antiretroviral therapy, treatment of HIV infection is not a realistic approach in these countries nor is likely to be in the foreseeable future. There is an urgent need to explore other approaches to control the epidemic, in particular preventative measures such as health education, treatment of sexually transmitted diseases, vaccines and topical microbicides.

There is a broad scientific consensus that a successful vaccine to prevent HIV-1 transmission must be able to elicit HIV-specific CD8+ cytotoxic T-lymphocytes (CTL) and also antibodies capable of neutralising primary HIV isolates (Nab). Major approaches toward this end include live, attenuated vaccines; inactivated viruses with adjuvants; subunit vaccines with adjuvants; live-vector based vaccines; and DNA vaccines. Major concerns regarding safety issues have been raised for the use of live, attenuated vaccines in humans. The protective immunity generated in monkeys immunized with inactivated viruses with adjuvants is not virus-specific. Subunit vaccines, such as highly purified recombinant monomeric HIV-1 envelope proteins elicit neither virus-specific CTL nor antibody responses that can neutralize primary patients isolates of HIV-1, even when adjuvanted with potent immunostimulants.

At the present, combining DNA vaccines and live-vector based vaccines in prime-boost regimens appears to be the most promising vaccine strategies. For instance, in one study, macaques primed with NYVAC-HIV1 env or NYVAC-HIV *env*/*gag-pol* and boosted with HIV-1 gp120 or peptide were protected against HIV2 challenge. In another study, macaques primed with NYVAC-HIV-2 *env*/*gag-pol* or NYVAC-HIV-2env and boosted with HIV-2 envelope have been protected against i.v. HIV-2 challenge. Ongoing studies in humans include a Phase I trial using DNA-prime (1mg or 2mg) and MVA-boost in 120 volunteers. There is a clear need in the art for effective immunological compositions for immunizing humans against HIV. Such compositions are provided by this disclosure.

The prior art includes J. Gen Virol., 85, 2004, 911-9. This paper discloses a protocol involving DNA priming and MVA boashing, and wherein in each case the antigens are a gag p24/p17 fused to a string of CTL epitopes.

### Brief Description of the Drawings

**Figure 1****.** Nucleotide sequence of NYVAC-HIV C plasmid
   (pMA60gp120C/gagpolnef-C-14.
**Figure 2****.** Percentage of responders following administration of NYVAC alone or DNA following by NYVAC (prime-boost).
**Figure 3****.** Measurement of INF-γ-secreting T cells following administration of NYVAC alone or DNA following by NYVAC (prime-boost).
**Figure 4****.** Difference in the magnitude of the immune following administration of NYVAC alone or DNA following by NYVAC (prime-boost).
**Figure 5****.** Representative flow cytometry profiles of env-specific INF-γ-secreting T cells following administration of NYVAC alone or DNA following by NYVAC (prime-boost).
**Figure 6****.** Correlation between the frequencies of INF-γ-secreting T cells measured by flow cytometry and ELISPOT.
**Figure 7****.** Flow cytometry profiles of CD4 and CD8 T cells recognizing various peptides following administration of NYVAC alone or DNA following by NYVAC (prime-boost).
**Figure** 8. IgG antibody levels at different time points following administration of NYVAC alone or DNA following by NYVAC (prime-boost).
**Figure 9**. Analysis of the immune response 72 weeks following administration of NYVAC alone or DNA following by NYVAC (prime-boost).

### Summary of the Invention

Disclosed herein are methods for immunizing human beings against HIV by inducing or enhancing a dominant CD4 T cell response against that agent.

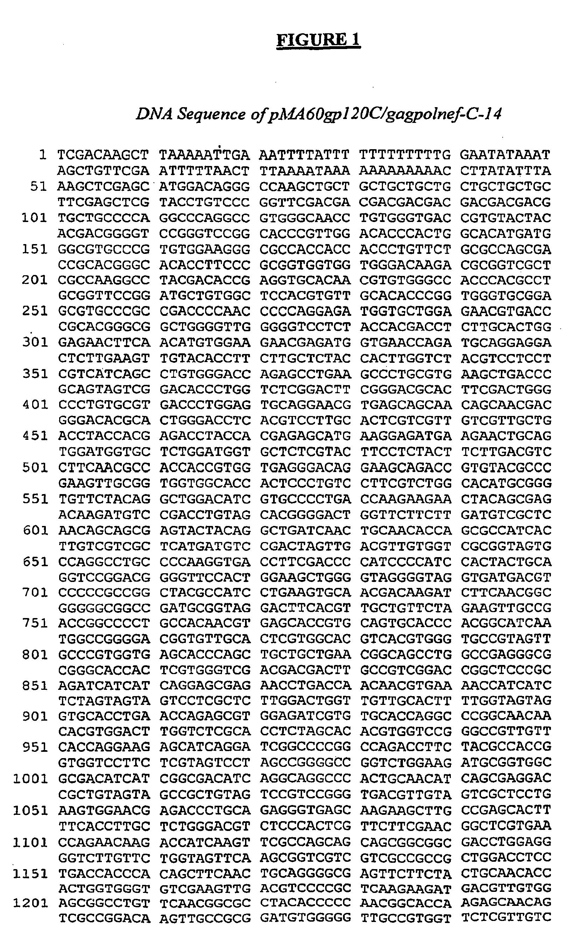

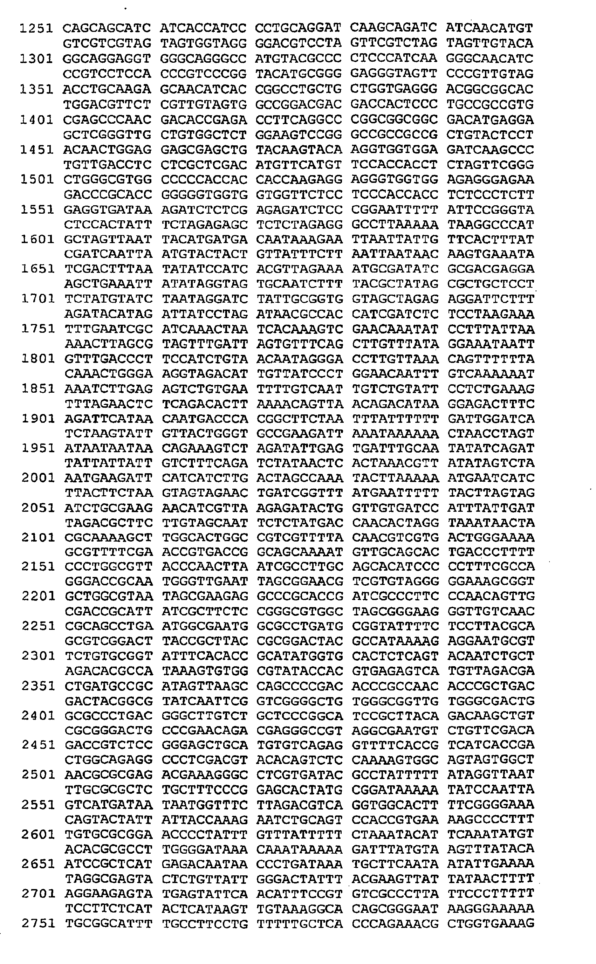

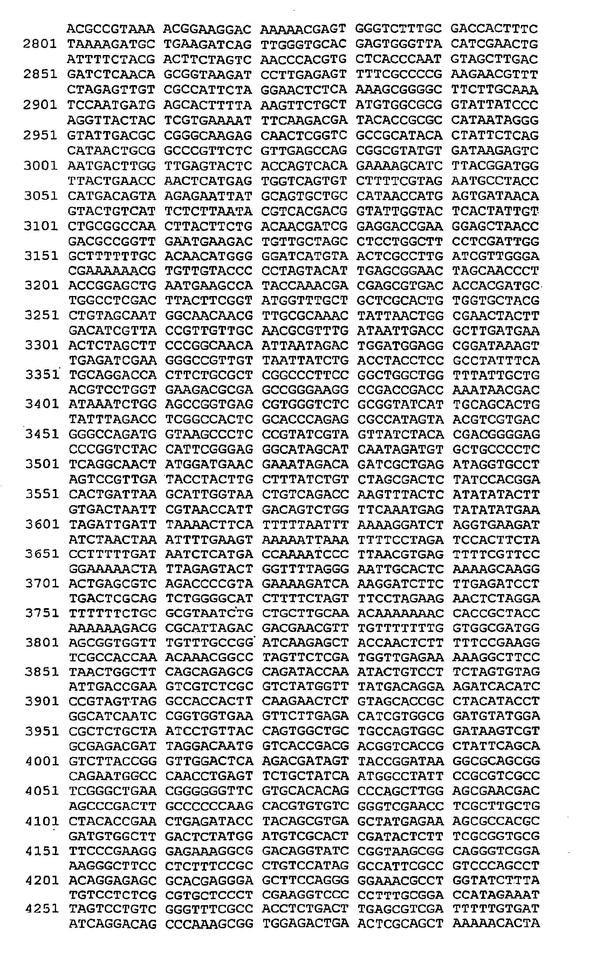

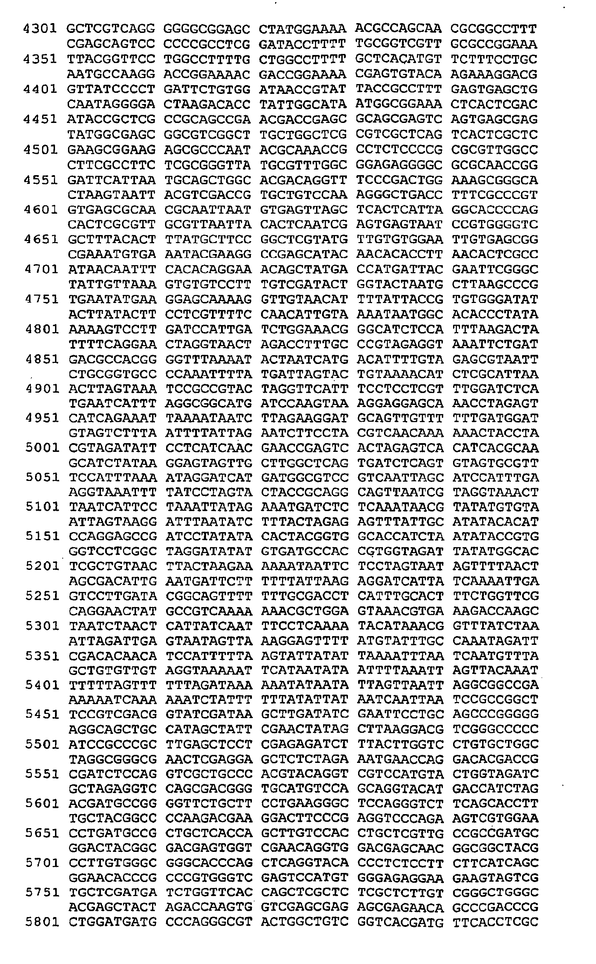

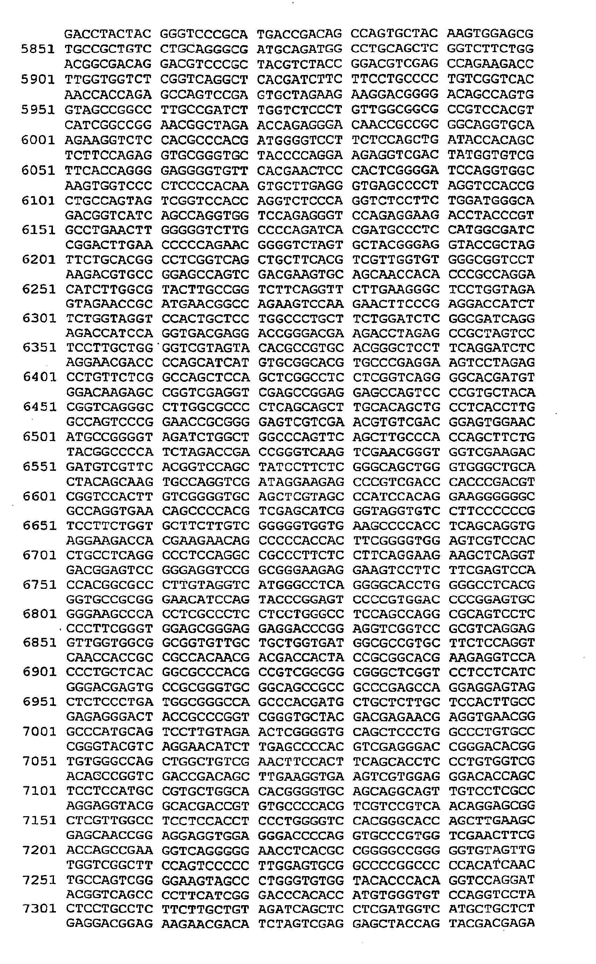

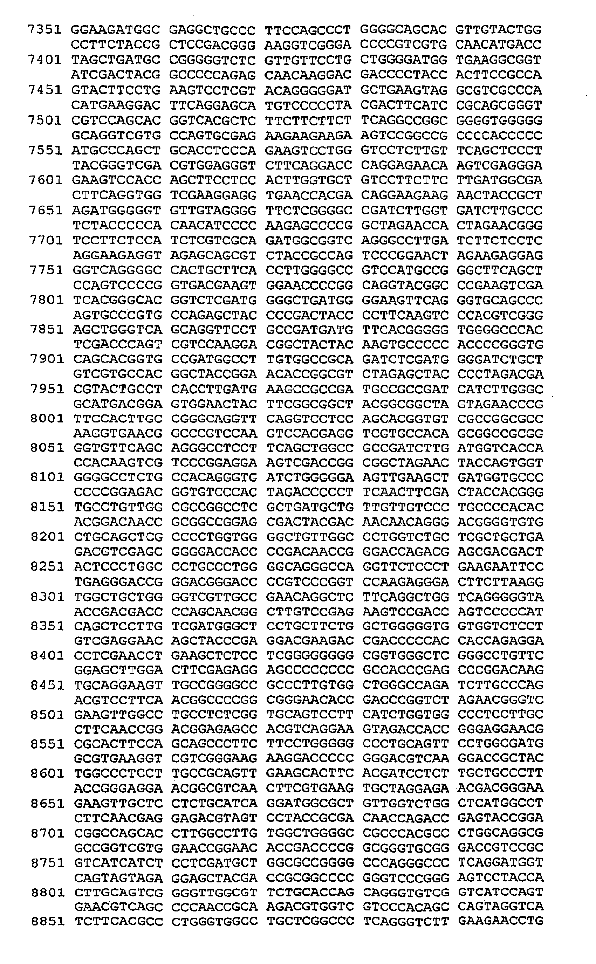

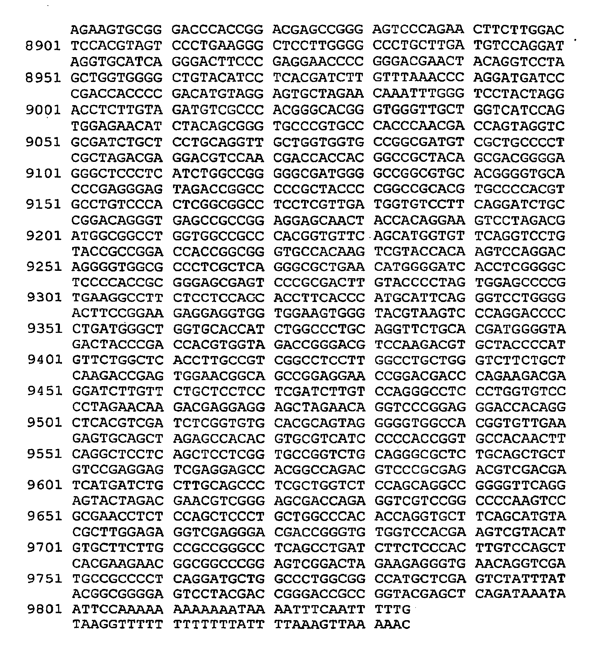

The invention is the following:
1. A two-part immunological composition for use in the treatment or prevention of human immunodeficiency virus (HIV) infection comprising a first form of an HIV immunogen and a second form of an HIV immunogen, the first forms being a DNA molecule and the second form being a NYVAC viral vector, each form encoding HIV immunogens comprising gp120, gag, pol and nef, wherein the forms are administered separately such that administration of the first form enhances an immune response against the subsequently administered second form relative to the administration of the second form alone, wherein the immune response comprises a dominant CD4 T cell response.
2. The composition for use of 1 wherein the HIV immunogen is encoded by a gagpolnef cassette of plasmid pMA60gp120C/gagpolnef -C-14 illustrated in Figure 1.
3. The composition for use of 1 or 2 wherein the HIV immunogen is encoded by the gagpolnef cassette of Figure 1.
4. The composition for use of any one of 1-3 wherein the immunogen is derived from HIV-1 intersubtype (C/B').
5. The composition for use of any one of 1-4 wherein the cellular response comprises polyfunctional CD4 T cells.
6. The composition for use of any one of 1-5 wherein the cellular response demonstrates at least two characteristics selected from the group consisting of:
   a. a high proportion of immunogen-specific CD4 cells within the population of total responding T cells;
   b. responding CD4 T cells that form up to about 1,000 or more spot-forming units (SFUs) by ELISPOT assay per one million blood mononuclear cells;
   c. polyfunctional CD4 T cells;
   d. responding CD4 T cells secret both IL-2 and IFN-γ; and
   e. the dominant CD4 T cell immune response encompasses at least two epitopes of an HIV immunogen.
7. The composition for use of any one of 1-6 wherein the cellular response further comprises CD8 cytotoxic T cells.
8. A composition for use of any one of 1-7 utilized in conjunction with at least one antiviral agent selected from the group consisting of an anti-HIV agent, a protease inhibitor, an HIV entry inhibitor, a reverse transcriptase inhibitor, and an anti- retroviral nucleoside analog, and wherein the at least one antiviral agent may be selected from the group consisting of Agenerase (amprenavir), Combivir, (Retrovir, Epivir), Crixivan (indinavir), Emtriva (emtricitabin), Epivir(3TC, lamivudine), Epzicom, Fortovase (Invirase, saquinavir), Fuzeon (enfuvirtide), Hivid (DDC, zalcitabine), Kaletra (lopinavir), Lexiva, (Fosamprenavir), Norvir(ritonsvir), Rescriptor(delavirdine), Retrovir(AZT, zidovudine), Reyatax(atazanavir, BMS-232632), Sustiva (efavirenz), Trizivir (abacavir, zidovudine, lamivudine), Truvada(Emtricitabine / Tenofovir DF), Videx (ddI, didanosine), Videx EC (didanosine) Viracept(nevirapine), Viread (tenofovir disoproxil fumarate), Zerit, (d4T, stavudine), and Ziagen ( abacavir).

### Detailed Description

The present invention provides compositions for treating and / or preventing HIV by stimulating an immune response against such an agent.

As used herein, an "immunogen" is a polypeptide, peptide or a portion or derivative thereof that produces an immune response in a host to whom the immunogen has been administered. The immunogen is typically isolated from its source (i.e., an infectious agent) of which it forms a part (i.e., a protein normally found within a cell). The immune response may include the production of antibodies that bind to at least one epitope of the immunogen and / or the generation of a cellular immune response against cells expressing an epitope of the immunogen. In certain cases the immunogen may be the epitope *per se.* Where different forms of immunogen are utilized, the immunogens may be the same or different. The immunogen may stimulate a *de novo* response or enhance an existing response against the immunogen by, for example, causing an increased antibody response (i.e., amount of antibody, increased affinity / avidity) or an increased cellular response (i.e., increased number of activated T cells, increased affinity / avidity of T cell receptors). In certain embodiments, the immune response is protective, meaning the immune response is capable of preventing infection of or growth within a host and / or by eliminating an agent (i.e., HIV) from a host.

The immunological compositions of the present disclosure may include one or more immunogen(s) from a single source or multiple sources. For instance, in certain embodiments the present disclosure relates to the induction or enhancement of an immune response against human immunodeficiency virus (HIV). Immunological compositions may include one or more immunogens expressed by cells infected with HIV and / or displayed on the HIV virion *per se.* With respect to HIV, the immunogens may be selected from any HIV isolate. As is well-known in the art, HIV isolates are now classified into discrete genetic subtypes. Subtype B has been associated with the HIV epidemic in homosexual men and intravenous drug users worldwide. Most immunogens, laboratory adapted isolates, reagents and mapped epitopes belong to subtype B. In sub-Saharan Africa, India and China, areas where the incidence of new HIV infections is high, subtype B accounts for only a small minority of infections, and subtype C appears to be the most common infecting subtype. Thus, in certain embodiments, it may be preferable to select immunogens from HIV subtypes B and / or C. It may be desirable to include immunogens from multiple HIV subtypes (i.e., HIV subtypes B and C) in a single immunological composition. Suitable HIV immunogens include ENV, GAG, POL, NEF, as well as variants, derivatives, and fusion proteins thereof, for example.

The present disclosure relates in certain embodiments to immunological compositions capable of inducing or enhancing a dominant CD4 T cell immune response against an immunogen. A dominant CD4 T cell immune response is typically characterized by observing high proportion of immunogen-specific CD4 cells within the population of total responding T cells following vaccination as determined by an IFN-γ ELISPOT assay. For example, this response may be characterized by the presence of up to 55; 100; 250; 500; 750; or 1,000 or more spot-forming units (SFUs) by IFN-γ ELISPOT assay per one million (10⁶) blood mononuclear cells. A dominant CD4 T cell immune response also typically but not necessarily provides a high proportion of responders (i.e., up to 50%, 60%, 70%, 80%, 85%, 90%, 95% or 100% of subjects tested) as compared to responders demonstrating a CD8 T cell immune response. A dominant CD4 T cell immune response is also typically but not necessarily polyfunctional, meaning that the majority of responding CD4 T cells secret both IL-2 and IFN-γ. A dominant CD4 T cell immune response also typically but not necessarily encompasses several epitopes (i.e., several populations of clonal CD4 T cells) within or between responders, as compared to mono-epitopic CD8 T cell responses. A dominant CD4 T cell response may include one, more than one or all of the characteristics described above. Surprisingly, it has been found that the immunological compositions and methods presented herein induce a dominant CD4 T cell response in human beings.

In preferred embodiments of the present disclosure, vectors are used to transfer a nucleic acid sequence encoding a polypeptide to a cell. A vector is any molecule used to transfer a nucleic acid sequence to a host cell. In certain cases, an expression vector is utilized. An expression vector is a nucleic acid molecule that is suitable for transformation of a host cell and contains nucleic acid sequences that direct and / or control the expression of the transferred nucleic acid sequences. Expression includes, but is not limited to, processes such as transcription, translation, and splicing, if introns are present. Expression vectors typically comprise one or more flanking sequences operably linked to a heterologous nucleic acid sequence encoding a polypeptide. As used herein, the term operably linked refers to a linkage between polynucleotide elements in a functional relationship such as one in which a promoter or enhancer affects transcription of a coding sequence. Flanking sequences may be homologous (i.e., from the same species and / or strain as the host cell), heterologous (i.e., from a species other than the host cell species or strain), hybrid (i.e., a combination of flanking sequences from more than one source), or synthetic, for example.

In certain embodiments, it is preferred that the flanking sequence is a trascriptional regulatory region that drives high-level gene expression in the target cell. The transcriptional regulatory region may comprise, for example, a promoter, enhancer, silencer, repressor element, or combinations thereof. The transcriptional regulatory region may be either constitutive, tissue-specific, cell-type specific (i.e., the region is drives higher levels of transcription in a one type of tissue or cell as compared to another), or regulatable (i.e., responsive to interaction with a compound such as tetracycline). The source of a transcriptional regulatory region may be any prokaryotic or eukaryotic organism, any vertebrate or invertebrate organism, or any plant, provided that the flanking sequence functions in a cell by causing transcription of a nucleic acid within that cell. A wide variety of transcriptional regulatory regions may be utilized in practicing the present invention.

Suitable transcriptional regulatory regions include, for example, the synthetic E/L promoter; the CMV promoter (i.e., the CMV-immediate early promoter); promoters from eukaryotic genes (i.e., the estrogen-inducible chicken ovalbumin gene, the interferon genes, the gluco-corticoid-inducible tyrosine aminotransferase gene, and the thymidine kinase gene); and the major early and late adenovirus gene promoters; the SV40 early promoter region (Bernoist and Chambon, 1981, Nature 290:304-10); the promoter contained in the 3' long terminal repeat (LTR) of Rous sarcoma virus (RSV) (Yamamoto, et al., 1980, Cell 22:787-97); the herpes simplex virus thymidine kinase (HSV-TK) promoter (Wagner et al., 1981, Proc. Natl. Acad. Sci. U.SA. 78:1444-45); the regulatory sequences of the metallothionine gene (Brinster et al., 1982, Nature 296:39-42); prokaryotic expression vectors such as the beta-lactamase promoter (Villa-Kamaroff et al., 1978, Proc. Natl. Acad Sci. U.S.A., 75:3727-31); or the tac promoter (DeBoer et al., 1983, Proc. Natl. Acad. Sci. U.S.A., 80:21-25). Tissue- and / or cell-type specific transcriptional control regions include, for example, the elastase I gene control region which is active in pancreatic acinar cells (Swift et al., 1984, Cell 38:639-46; Ornitz et al., 1986, Cold Spring Harbor Symp. Quant. Biol. 50:399-409 (1986); MacDonald, 1987, Hepatology 7:425-515); the insulin gene control region which is active in pancreatic beta cells (Hanahan, 1985, Nature 315:115-22); the immunoglobulin gene control region which is active in lymphoid cells (Grosschedl et al., 1984, Cell 38:647-58; Adames et al., 1985, Nature 318:533-38; Alexander et al., 1987, Mol. Cell. Biol., 7:1436-44); the mouse mammary tumor virus control region in testicular, breast, lymphoid and mast cells (Leder et al., 1986, Cell 45:485-95); the albumin gene control region in liver (Pinkert et al., 1987, Genes and Devel. 1:268-76); the alpha-feto-protein gene control region in liver (Krumlauf et al., 1985, Mol. Cell. Biol., 5:1639-48; Hammer et al., 1987, Science 235:53-58); the alpha 1-antitrypsin gene control region in liver (Kelsey et al., 1987, Genes and Devel. 1:161-71); the beta-globin gene control region in myeloid cells (Mogram et al., 1985, Nature 315:338-40; Kollias et al., 1986, Cell 46:89-94); the myelin basic protein gene control region in oligodendrocyte cells in the brain (Readhead et al., 1987, Cell 48:703-12); the myosin light chain-2 gene control region in skeletal muscle (Sani, 1985, Nature 314:283-86); the gonadotropic releasing hormone gene control region in the hypothalamus (Mason et al., 1986, Science 234:1372-78), and the tyrosinase promoter in melanoma cells (Hart, I. Semin Oncol 1996 Feb;23(1):154-8; Siders, et al. Cancer Gene Ther 1998 Sep-Oct;5(5):281-91), among others. Other suitable promoters are known in the art.

In certain embodiments, a substitution of one amino acid for another may be made in the sequence of an immunogen. Substitutions may be conservative, or non-conservative, or any combination thereof. Conservative amino acid modifications to the sequence of a polypeptide (and the corresponding modifications to the encoding nucleotides) may produce polypeptides having functional and chemical characteristics similar to those of a parental polypeptide. For example, a "conservative amino acid substitution" may involve a substitution of a native amino acid residue with a non-native residue such that there is little or no effect on the size, polarity, charge, hydrophobicity, or hydrophilicity of the amino acid residue at that position and, in particlar, does not result in decreased immunogenicity. Suitable substitutions may be selected from the following T**able I**:

**Table I**

| Original Residues | Exemplary Substitutions | Preferred Substitutions |
|---|---|---|
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

In other embodiments, it may be advantageous to combine a nucleic acid sequence encoding an immunogen with one or more co-stimulatory component(s) such as cell surface proteins, cytokines or chemokines in a composition of the present invention. The co-stimulatory component may be included in the composition as a polypeptide or as a nucleic acid encoding the polypeptide, for example. Suitable co-stimulatory molecules include, for instance, polypeptides that bind members of the CD28 family (i.e., CD28, ICOS; Hutloff, et al. Nature 1999, 397: 263-265; Peach, et al. J Exp Med 1994, 180: 2049-2058) such as the CD28 binding polypeptides B7.1 (CD80; Schwartz, 1992; Chen et al, 1992; Ellis, et al. J. Immunol., 156(8): 2700-9) and B7.2 (CD86; Ellis, et al. J. Immunol., 156(8): 2700-9); polypeptides which bind members of the integrin family (i.e., LFA-1 (CD11a / CD18); Sedwick, et al. J Immunol 1999, 162: 1367-1375; Wülfing, et al. Science 1998, 282: 2266-2269; Lub, et al. Immunol Today 1995, 16: 479-483) including members of the ICAM family (i.e., ICAM-1, -2 or -3); polypeptides which bind CD2 family members (i.e., CD2, signalling lymphocyte activation molecule (CDw150 or "SLAM"; Aversa, et al. J Immunol 1997, 158: 4036-4044) such as CD58 (LFA-3; CD2 ligand; Davis, et al. Immunol Today 1996, 17: 177-187) or SLAM ligands (Sayos, et al. Nature 1998, 395: 462-469); polypeptides which bind heat stable antigen (HSA or CD24; Zhou, et al. Eur J Immunol 1997, 27: 2524-2528); polypeptides which bind to members of the TNF receptor (TNFR) family (i.e., 4-1BB (CD137; Vinay, et al. Semin Immunol 1998, 10: 481-489)), OX40 (CD 134; Weinberg, et al. Semin Immunol 1998, 10: 471-480; Higgins, et al. J Immunol 1999, 162: 486-493), and CD27 (Lens, et al. Semin Immunol 1998, 10: 491-499)) such as 4-1BBL (4-1BB ligand; Vinay, et al. Semin Immunol 1998, 10: 481-48; DeBenedette, et al. J Immunol 1997, 158: 551-559), TNFR associated factor-1 (TRAF-1; 4-1BB ligand; Saoulli, et al. J Exp Med 1998, 187: 1849-1862, Arch, et al. Mol Cell Biol 1998, 18: 558-565), TRAF-2 (4-1BB and OX40 ligand; Saoulli, et al. J Exp Med 1998, 187: 1849-1862; Oshima, et al. Int Immunol 1998, 10: 517-526, Kawamata, et al. J Biol Chem 1998, 273: 5808-5814), TRAF-3 (4-1 BB and OX40 ligand; Arch, et al. Mol Cell Biol 1998, 18: 558-565; Jang, et al. Biochem Biophys Res Commun 1998, 242: 613-620; Kawamata S, et al. J Biol Chem 1998, 273: 5808-5814), OX40L (OX40 ligand; Gramaglia, et al. J Immunol 1998, 161: 6510-6517), TRAF-5 (OX40 ligand; Arch, et al. Mol Cell Biol 1998, 18: 558-565; Kawamata, et al. J Biol Chem 1998, 273: 5808-5814), and CD70 (CD27 ligand; Couderc, et al. Cancer Gene Ther., 5(3): 163-75). CD154 (CD40 ligand or "CD40L"; Gurunathan, et al. J. Immunol., 1998, 161: 4563-4571; Sine, et al. Hum. Gene Ther., 2001, 12: 1091-1102) Other co-stimulatory molecules may also be suitable for practicing the present invention.

One or more cytokines may also be suitable co-stimulatory components or "adjuvants", either as polypeptides or being encoded by nucleic acids contained within the compositions of the present invention (Parmiani, et al. Immunol Lett 2000 Sep 15; 74(1): 41-4; Berzofsky, et al. Nature Immunol. 1: 209-219). Suitable cytokines include, for example, interleukin-2 (IL-2) (Rosenberg, et al. Nature Med 4: 321-327 (1998)), IL-4, IL-7, IL-12 (reviewed by Pardoll, 1992; Harries, et al. J. Gene Med. 2000 Jul-Aug;2(4):243-9; Rao, et al. J. Immunol. 156: 3357-3365 (1996)), IL-15 (Xin, et al. Vaccine, 17:858-866, 1999), IL-16 (Cruikshank, et al. J. Leuk Biol. 67(6): 757-66, 2000), IL-18 (J. Cancer Res. Clin. Oncol. 2001. 127(12): 718-726), GM-CSF (CSF (Disis, et al. Blood, 88: 202-210 (1996)), tumor necrosis factor-alpha (TNF-α), or interferon-gamma (INF-γ). Other cytokines may also be suitable for practicing the present invention.

Chemokines may also be utilized. For example, fusion proteins comprising CXCL10 (IP-10) and CCL7 (MCP-3) fused to a tumor self-antigen have been shown to induce anti-tumor immunity (Biragyn, et al. Nature Biotech. 1999, 17: 253-258). The chemokines CCL3 (MIP-1α) and CCL5 (RANTES) (Boyer, et al. Vaccine, 1999, 17 (Supp. 2): S53-S64) may also be of use in practicing the present invention. Other suitable chemokines are known in the art.

It is also known in the art that suppressive or negative regulatory immune mechanisms may be blocked, resulting in enhanced immune responses. For instance, treatment with anti-CTLA-4 (Shrikant, et al. Immunity, 1996, 14: 145-155; Sutmuller, et al. J. Exp. Med., 2001, 194: 823-832), anti-CD25 (Sutmuller, *supra*), anti-CD4 (Matsui, et al, J. Immunol., 1999, 163: 184-193), the fusion protein IL13Ra2-Fc (Terabe, et al. Nature Immunol., 2000, 1: 515-520), and combinations thereof (i.e., anti-CTLA-4 and anti-CD25, Sutmuller, *supra*) have been shown to upregulate anti-tumor immune responses and would be suitable in practicing the present invention.

An immunogen may also be administered in combination with one or more adjuvants to boost the immune response. Adjuvants may also be included to stimulate or enhance the immune response against PhtD. Non-limiting examples of suitable adjuvants include those of the gel-type (i.e., aluminum hydroxide/phosphate ("alum adjuvants"), calcium phosphate), of microbial origin (muramyl dipeptide (MDP)), bacterial exotoxins (cholera toxin (CT), native cholera toxin subunit B (CTB), E. coli labile toxin (LT), pertussis toxin (PT), CpG oligonucleotides, BCG sequences, tetanus toxoid, monophosphoryl lipid A (MPL) of, for example, *E*. *coli, Salmonella minnesota, Salmonella typhimurium,* or *Shigella exseri),* particulate adjuvants (biodegradable, polymer microspheres), immunostimulatory complexes (ISCOMs)), oil-emulsion and surfactant-based adjuvants (Freund's incomplete adjuvant (FIA), microfluidized emulsions (MF59, SAF), saponins (QS-21)), synthetic (muramyl peptide derivatives (murabutide, threony-MDP), nonionic block copolymers (L121), polyphosphazene (PCCP), synthetic polynucleotides (poly A :U, poly I :C), thalidomide derivatives (CC-4407/ACTIMID)), RH3-ligand, or polylactide glycolide (PLGA) microspheres, among others. Fragments, homologs, derivatives, and fusions to any of these toxins are also suitable, provided that they retain adjuvant activity. Suitable mutants or variants of adjuvants are described, e.g., in WO 95/17211 (Arg-7- Lys CT mutant), WO 96/6627 (Arg-192-Gly LT mutant), and WO 95/34323 (Arg-9-Lys and Glu-129-Gly PT mutant). Additional LT mutants that can be used in the methods and compositions of the invention include, e. g., Ser-63-Lys, Ala-69-Gly,Glu-110-Asp, and Glu-112-Asp mutants. Other suitable adjuvants are also well-known in the art.

As an example, metallic salt adjuvants such alum adjuvants are well-known in the art as providing a safe excipient with adjuvant activity. The mechanism of action of these adjuvants are thought to include the formation of an antigen depot such that antigen may stay at the site of injection for up to 3 weeks after administration, and also the formation of antigen/metallic salt complexes which are more easily taken up by antigen presenting cells. In addition to aluminium, other metallic salts have been used to adsorb antigens, including salts of zinc, calcium, cerium, chromium, iron, and berilium. The hydroxide and phosphate salts of aluminium are the most common. Formulations or compositions containing aluminium salts, antigen, and an additional immunostimulant are known in the art. An example of an immunostimulant is 3-de-O-acylated monophosphoryl lipid A (3D-MPL).

Any of these components may be used alone or in combination with other agents. For instance, it has been shown that a combination of CD80, ICAM-1 and LFA-3 ("TRICOM") may potentiate anti-cancer immune responses (Hodge, et al. Cancer Res. 59: 5800-5807 (1999). Other effective combinations include, for example, IL-12 + GM-CSF (Ahlers, et al. J. Immunol., 158: 3947-3958 (1997); Iwasaki, et al. J. Imnmnol. 158: 4591-4601 (1997)), IL-12 + GM-CSF + TNF-α (Ahlers, et al. Int. Immunol. 13: 897-908 (2001)), CD80 + IL-12 (Fruend, et al. Int. J. Cancer, 85: 508-517 (2000); Rao, et al. *supra*), and CD86 + GM-CSF + IL-12 (Iwasaki, supra). One of skill in the art would be aware of additional combinations useful in carrying out the present disclosure. In addition, the skilled artisan would be aware of additional reagents or methods that may be used to modulate such mechanisms. These reagents and methods, as well as others known by those of skill in the art, may be utilized in practicing the present disclosure.

Other agents that may be utilized in conjunction with the compositions and methods provided herein include anti-HIV agents including, for example, protease inhibitor, an HIV entry inhibitor, a reverse transcriptase inhibitor, and / or or an antiretroviral nucleoside analog. Suitable compounds include, for example, Agenerase (amprenavir), Combivir (Retrovir / Epivir), Crixivan (indinavir), Emtriva (emtricitabine), Epivir (3tc / lamivudine), Epzicom, Fortovase / Invirase (saquinavir), Fuzeon (enfuvirtide), Hivid (ddc / zalcitabine), Kaletra (lopinavir), Lexiva (Fosamprenavir), Norvir (ritonavir), Rescriptor (delavirdine), Retrovir / AZT (zidovudine), Reyatax (atazanavir, BMS-232632), Sustiva (efavirenz), Trizivir (abacavir / zidovudine / lamivudine), Truvada (Emtricitabine / Tenofovir DF), Videx (ddI / didanosine), Videx EC (ddI, didanosine), Viracept (nevirapine), Viread (tenofovir disoproxil fumarate), Zerit (d4T / stavudine), and Ziagen (abacavir). Other suitable agents are known to those of skill in the art. Such agents may either be used prior to, during, or after administration of the compositions and / or use of the methods described herein.

Nucleic acids encoding immunogens may be administered to patients by any of several available techniques. Various viral vectors that have been successfully utilized for introducing a nucleic acid to a host include retrovirus, adenovirus, adeno-associated virus (AAV), alphavirus, herpes virus, and poxvirus, among others. It is understood in the art that many such viral vectors are available in the art. The vectors of the present invention may be constructed using standard recombinant techniques widely available to one skilled in the art. Such techniques may be found in common molecular biology references such as Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), and PCR Protocols: A Guide to Methods and Applications (Innis, et al. 1990. Academic Press, San Diego, CA).

Preferred retroviral vectors are derivatives of lentivirus as well as derivatives of murine or avian retroviruses. Examples of suitable retroviral vectors include, for example, Moloney murine leukemia virus (MoMuLV), Harvey murine sarcoma virus (HaMuSV), murine mammary tumor virus (MuMTV), SIV, BIV, HIV and Rous Sarcoma Virus (RSV). A number of retroviral vectors can incorporate multiple exogenous nucleic acid sequences. As recombinant retroviruses are defective, they require assistance in order to produce infectious vector particles. This assistance can be provided by, for example, helper cell lines encoding retrovirus structural genes. Suitable helper cell lines include Ψ2, PA317 and PA12, among others. The vector virions produced using such cell lines may then be used to infect a tissue cell line, such as NIH 3T3 cells, to produce large quantities of chimeric retroviral virions. Retroviral vectors may be administered by traditional methods (i.e., injection) or by implantation of a "producer cell line" in proximity to the target cell population (Culver, K., et al., 1994, Hum. Gene Ther., 5 (3): 343-79; Culver, K., et al., Cold Spring Harb. Symp. Quant. Biol., 59: 685-90); Oldfield, E., 1993, Hum. Gene Ther., 4 (1): 39-69). The producer cell line is engineered to produce a viral vector and releases viral particles in the vicinity of the target cell. A portion of the released viral particles contact the target cells and infect those cells, thus delivering a nucleic acid encoding an immunogen to the target cell. Following infection of the target cell, expression of the nucleic acid of the vector occurs.

Adenoviral vectors have proven especially useful for gene transfer into eukaryotic cells (Rosenfeld, M., et al., 1991, Science, 252 (5004): 431-4; Crystal, R., et al., 1994, Nat. Genet., 8 (1): 42-51), the study eukaryotic gene expression (Levrero, M., et al., 1991, Gene, 101 (2): 195-202), vaccine development (Graham, F. and Prevec, L., 1992, Biotechnology, 20: 363-90), and in animal models (Stratford-Perricaudet, L., et al., 1992, Bone Marrow Transplant., 9 (Suppl. 1): 151-2 ; Rich, D., et al., 1993, Hum. Gene Ther., 4 (4): 461-76). Experimental routes for administrating recombinant Ad to different tissues *in vivo* have included intratracheal instillation (Rosenfeld, M., et al., 1992, Cell, 68 (1): 143-55) injection into muscle (Quantin, B., et al., 1992, Proc. Natl. Acad. Sci. U.S.A., 89 (7): 2581-4), peripheral intravenous injection (Herz, J., and Gerard, R., 1993, Proc. Natl. Acad. Sci. U.S.A., 90 (7): 2812-6) and stereotactic inoculation to brain (Le Gal La Salle, G., et al., 1993, Science, 259 (5097): 988-90), among others.

Adeno-associated virus (AAV) demonstrates high-level infectivity, broad host range and specificity in integrating into the host cell genome (Hermonat, P., et al., 1984, Proc. Natl. Acad. Sci. U.S.A., 81 (20): 6466-70). And Herpes Simplex Virus type-1 (HSV-1) is yet another attractive vector system, especially for use in the nervous system because of its neurotropic property (Geller, A., et al., 1991, Trends Neurosci., 14 (10): 428-32; Glorioso, et al., 1995, Mol. Biotechnol., 4 (1): 87-99; Glorioso, et al., 1995, Annu. Rev. Microbiol., 49: 675-7 10).

Alphavirus may also be used to express the immunogen in a host. Suitable members of the Alphavirus genus include, among others, Sindbis virus, Semliki Forest virus (SFV), the Ross River virus and Venezuelan, Western and Eastern equine encephalitis viruses, among others. Expression systems utilizing alphavirus vectors are described in, for example, U.S. Pat. Nos. 5,091,309; 5,217,879; 5,739,026; 5,766,602; 5,843,723; 6,015,694; 6,156,558; 6,190,666; 6,242,259; and, 6,329,201; WO 92/10578; Xiong et al., Science; Vol 243, 1989, 1188-1191; Liliestrom, et al. Bio/Technology, 9: 1356-1361, 1991. Thus, the use of alphavirus as an expression system is well known by those of skill in the art.

Poxvirus is another useful expression vector (Smith, et al. 1983, Gene, 25 (1): 21-8; Moss, et al, 1992, Biotechnology, 20: 345-62; Moss, et al, 1992, Curr. Top. Microbiol. Immunol., 158: 25-38; Moss, et al. 1991. Science, 252: 1662-1667). The most often utilized poxviral vectors include vaccinia and derivatives therefrom such as NYVAC and MVA, and members of the avipox genera such as fowlpox, canarypox, ALVAC, and ALVAC(2), among others.

An exemplary suitable vector is NYVAC (vP866) which was derived from the Copenhagen vaccine strain of vaccinia virus by deleting six nonessential regions of the genome encoding known or potential virulence factors (see, for example, U.S. Pat. Nos. 5,364,773 and 5,494,807). The deletion loci were also engineered as recipient loci for the insertion of foreign genes. The deleted regions are: thymidine kinase gene (TK; J2R); hemorrhagic region (u; B13R+B14R); A type inclusion body region (ATI; A26L); hemagglutinin gene (HA; A56R); host range gene region (C7L-K1L); and, large subunit, ribonucleotide reductase (I4L). NYVAC is a genetically engineered vaccinia virus strain that was generated by the specific deletion of eighteen open reading frames encoding gene products associated with virulence and host range. NYVAC has been show to be useful for expressing TAs (see, for example, U.S. Pat. No. 6,265,189). NYVAC (vP866), vP994, vCP205, vCP1433, placZH6H4Lreverse, pMPC6H6K3E3 and pC3H6FHVB were also deposited with the ATCC under the terms of the Budapest Treaty, accession numbers VR-2559, VR-2558, VR-2557, VR-2556, ATCC-97913, ATCC-97912, and ATCC-97914, respectively.

Another suitable virus is the Modified Vaccinia Ankara (MVA) virus which was generated by 516 serial passages on chicken embryo fibroblasts of the Ankara strain of vaccinia virus (CVA) (for review see Mayr, A., et al. Infection 3, 6-14 (1975)). It was shown in a variety of animal models that the resulting MVA was significantly avirulent (Mayr, A. & Danner, K. [1978] Dev. Biol. Stand. 41: 225.34) and has been tested in clinical trials as a smallpox vaccine (Mayr et al., Zbl. Bakt. Hyg. I, Abt. Org. B 167, 375-390 (1987), Stickl et al., Dtsch. med. Wschr. 99, 2386-2392 (1974)). MVA has also been engineered for use as a viral vector for both recombinant gene expression studies and as a recombinant vaccine (Sutter, G. et al. (1994), Vaccine 12: 1032-40; Blanchard et al., 1998, J Gen Virol 79, 159-1167; Carroll & Moss, 1997, Virology 238, 198-211; Altenberger, U.S. Pat. No. 5,185,146; Ambrosini et al., 1999, J Neurosci Res 55(5), 569).

ALVAC-based recombinant viruses (i.e., ALVAC-1 and ALVAC-2) are also suitable for use in practicing the present invention (see, for example, U.S. Pat. No. 5,756,103). ALVAC(2) is identical to ALVAC(1) except that ALVAC(2) genome comprises the vaccinia E3L and K3L genes under the control of vaccinia promoters (U.S. Pat. No. 6,130,066; Beattie et al., 1995a, 1995b, 1991; Chang et al., 1992; Davies et al., 1993). Both ALVAC(1) and ALVAC(2) have been demonstrated to be useful in expressing foreign DNA sequences, such as TAs (Tartaglia et al., 1993 a,b; U.S. Pat. No. 5,833,975). ALVAC was deposited under the terms of the Budapest Treaty with the American Type Culture Collection (ATCC), 10801 University Boulevard, Manassas, Va. 20110-2209, USA, ATCC accession number VR-2547.

Another useful poxvirus vector is TROVAC. TROVAC refers to an attenuated fowlpox that was a plaque-cloned isolate derived from the FP-1 vaccine strain of fowlpoxvirus which is licensed for vaccination of 1 day old chicks. TROVAC was likewise deposited under the terms of the Budapest Treaty with the ATCC, accession number 2553.

"Non-viral" plasmid vectors may also be suitable in practicing the present disclosure. Plasmid DNA molecules comprising expression cassettes for expressing an immunogen may be used for "naked DNA" immunization. Preferred plasmid vectors are compatible with bacterial, insect, and / or mammalian host cells. Such vectors include, for example, PCR-II, pCR3, and pcDNA3.1 (Invitrogen, San Diego, CA), pBSII (Stratagene, La Jolla, CA), pET15 (Novagen, Madison, WI), pGEX (Pharmacia Biotech, Piscataway, NJ), pEGFP-N2 (Clontech, Palo Alto, CA), pETL (BlueBacII, Invitrogen), pDSR-alpha (PCT pub. No. WO 90/14363) and pFastBacDual (Gibco-BRL, Grand Island, NY) as well as Bluescript^{®} plasmid derivatives (a high copy number COLEI-based phagemid, Stratagene Cloning Systems, La Jolla, CA), PCR cloning plasmids designed for cloning Taq-amplified PCR products (*e.g*., TOPO™ TA cloning^{®} kit, PCR2.1^{®} plasmid derivatives, Invitrogen, Carlsbad, CA).

Bacterial vectors may also be used with the current disclosure. These vectors include, for example, *Shigella, Salmonella, Vibrio cholerae, Lactobacillus, Bacille calmette guérin (BCG), and Streptococcus* (see for example, WO 88/6626; WO 90/0594; WO 91/13157; WO 92/1796; and WO 92/21376). Many other non-viral plasmid expression vectors and systems are known in the art and could be used with the current disclosure.

Additional nucleic acid delivery techniques include DNA-ligand complexes, adenovirus-ligand-DNA complexes, direct injection of DNA, CaPO₄ precipitation, gene gun techniques, electroporation, and colloidal dispersion systems, among others. Colloidal dispersion systems include macromolecule complexes, nanocapsules, microspheres, beads, and lipid-based systems including oil-in-water emulsions, micelles, mixed micelles, and liposomes. The preferred colloidal system of this disclosure is a liposome, which are artificial membrane vesicles useful as delivery vehicles *in vitro* and *in vivo.* RNA, DNA and intact virions can be encapsulated within the aqueous interior and be delivered to cells in a biologically active form (Fraley, R., et al., 1981, Trends Biochem. Sci., 6: 77). The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations. Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

Strategies for improving the efficiency of nucleic acid-based immunization may also be used including, for example, the use of self-replicating viral replicons (Caley, et al. 1999. Vaccine, 17: 3124-2135; Dubensky, et al. 2000. Mol. Med. 6: 723-732; Leitner, et al. 2000. Cancer Res. 60: 51-55), codon optimization (Liu, et al. 2000. Mol. Ther., 1: 497-500; Dubensky, *supra;* Huang, et al. 2001. J. Virol. 75: 4947-4951*), in vivo* electroporation (Widera, et al. 2000, J. Immunol. 164: 4635-3640), incorporation of CpG stimulatory motifs (Gurunathan, et al. Ann. Rev. Immunol., 2000, 18: 927-974; Leitner, *supra),* sequences for targeting of the endocytic or ubiquitin-processing pathways (Thomson, et al. 1998. J. Virol. 72: 2246-2252; Velders, et al. 2001. J. Immunol. 166: 5366-5373), prime-boost regimens (Gurunathan, *supra;* Sullivan, et al. 2000. Nature, 408: 605-609; Hanke, et al. 1998. Vaccine, 16: 439-445; Amara, et al. 2001. Science, 292: 69-74), and the use of mucosal delivery vectors such as *Salmonella* (Darji, et al. 1997. Cell, 91: 765-775; Woo, et al. 2001. Vaccine, 19: 2945-2954). Other methods are known in the art, some of which are described bellow.

Administration of a composition of the present disclosure to a host may be accomplished using any of a variety of techniques known to those of skill in the art. The composition(s) may be processed in accordance with conventional methods of pharmacy to produce medicinal agents for administration to patients, including humans and other mammals (i.e., a "pharmaceutical composition"). The pharmaceutical composition is preferably made in the form of a dosage unit containing a given amount of DNA, viral vector particles, polypeptide, peptide, or other drug candidate, for example. A suitable daily dose for a human or other mammal may vary widely depending on the condition of the patient and other factors, but, once again, can be determined using routine methods. The compositions are administered to a patient in a form and amount sufficient to elicit a therapeutic effect, i.e., to induce a dominant CD4 T cell response. Amounts effective for this use will depend on various factors, including for example, the particular composition of the vaccine regimen administered, the manner of administration, the stage and severity of the disease, the general state of health of the patient, and the judgment of the prescribing physician. The dosage regimen for immunizing a host or otherwise treating a disorder or a disease with a composition of this invention is based on a variety of factors, including the type of disease, the age, weight, sex, medical condition of the patient, the severity of the condition, the route of administration, and the particular compound employed. Thus, the dosage regimen may vary widely, but can be determined routinely using standard methods.

In general, recombinant viruses may be administered in compositions in an amount of about 10⁴ to about 10⁹ pfu per inoculation; often about 10⁴ pfu to about 10⁶ pfu. Higher dosages such as about 10⁴ pfu to about 10¹⁰ pfu, e.g., about 10⁵ pfu to about 16⁹ pfu, or about 10⁶ pfu to about 10⁸ pfu, or about 10⁷ pfu can also be employed. Another measure commonly used is DICC₅₀; suitable DICC₅₀ ranges for administration include about 10¹, about 10², about 10³, about 10⁴, about 10⁵, about 10⁶, about 10⁷, about 10⁸, about 10⁹, about 10¹⁰ DICC₅₀. Ordinarily, suitable quantities of plasmid or naked DNA are about 1 µg to about 100 mg, about 1 mg, about 2 mg, but lower levels such as 0.1 to 1 mg or 1-10 µg may be employed. Actual dosages of such compositions can be readily determined by one of ordinary skill in the field of vaccine technology.

The pharmaceutical composition may be administered orally, parentally, by inhalation spray, rectally, intranodally, or topically in dosage unit formulations containing conventional pharmaceutically acceptable carriers, adjuvants, and vehicles. The term "pharmaceutically acceptable carrier" or "physiologically acceptable carrier" as used herein refers to one or more formulation materials suitable for accomplishing or enhancing the delivery of a nucleic acid, polypeptide, or peptide as a pharmaceutical composition. A "pharmaceutical composition" is a composition comprising a therapeutically effective amount of a nucleic acid or polypeptide. The terms "effective amount" and "therapeutically effective amount" each refer to the amount of a nucleic acid or polypeptide used to induce or enhance a dominant CD4 T cell response.

Injectable preparations, such as sterile injectable aqueous or oleaginous suspensions, may be formulated according to known methods using suitable dispersing or wetting agents and suspending agents. The injectable preparation may also be a sterile injectable solution or suspension in a non-toxic parenterally acceptable diluent or solvent. Suitable vehicles and solvents that may be employed are water, Ringer's solution, and isotonic sodium chloride solution, among others. For instance, a viral vector such as a poxvirus may be prepared in 0.4% NaCl or a Tris-HCl buffer, with or without a suitable stabilizer such as lactoglutamate, and with or without freeze drying medium. In addition, sterile, fixed oils are conventionally employed as a solvent or suspending medium. For this purpose, any bland fixed oil may be employed, including synthetic mono- or diglycerides. In addition, fatty acids such as oleic acid find use in the preparation of injectables.

Pharmaceutical compositions comprising a nucleic acid, immunogen(s), or other compound may take any of several forms and may be administered by any of several routes. In preferred embodiments, the compositions are administered via a parenteral route (intradermal, intramuscular or subcutaneous) to induce an immune response in the host. Alternatively, the composition may be administered directly into a lymph node (intranodal) or tumor mass (i.e., intratumoral administration).

Preferred embodiments of administratable compositions include, for example, nucleic acids, viral particles, or polypeptides in liquid preparations such as suspensions, syrups, or elixirs. Preferred injectable preparations include, for example, nucleic acids or polypeptides suitable for parental, subcutaneous, intradermal, intramuscular or intravenous administration such as sterile suspensions or emulsions. For example, a naked DNA molecule and / or recombinant poxvirus may separately or together be in admixture with a suitable carrier, diluent, or excipient such as sterile water, physiological saline, glucose or the like. The composition may also be provided in lyophilized form for reconstituting, for instance, in isotonic aqueous, saline buffer. In addition, the compositions can be co-administered or sequentially administered with one another, other antiviral compounds, other anti-cancer compounds and/or compounds that reduce or alleviate ill effects of such agents.

As previously mentioned, while the compositions of the disclosure can be administered as the sole active pharmaceutical agent, they can also be used in combination with one or more other compositions or agents (i.e., other immunogens, co-stimulatory molecules, adjuvants). When administered as a combination, the individual components can be formulated as separate compositions administered at the same time or different times, or the components can be combined as a single composition.

A kit comprising a composition of the present disclosure is also provided. The kit can include a separate container containing a suitable carrier, diluent or excipient. The kit can also include an additional components for simultaneous or sequential-administration. In one embodiment, such a kit may include a first form of an immunogen and a second form of the immunogen. Additionally, the kit can include instructions for mixing or combining ingredients and/or administration. A kit may provide reagents for performing screening assays, such as one or more PCR primers, hybridization probes, and / or biochips, for example.

A better understanding of the present invention and of its many advantages will be had from the following examples, given by way of illustration.

### EXAMPLES

### Example 1

### Materials and Methods

The recombinant vectors DNA C and NYVAC-HIV C expressed HIV genes derived from the Chinese R5 clade C virus (97CN54; Su, et al. J. Virol. 2000. 74: 11367-76; WO 01/36614). This clone has been shown to be representative of clade C strains circulating in China and India. All HIV genes have been optimised for codon usage since it has recently been shown that humanization of synthetic HIV gene codons allowed for an enhanced and REV/RRE- independent expression of *env* and *gag-pol* genes in mammalian cells. Genes were optimised for both safety and translation efficiency. The env gene has been designed to express the secreted gp 120 form of the envelope proteins and contain an optimal synthetic leader sequence for enhanced expression. The *gag, pol* and *nef* genes were fused to express a GAG-POL-NEF polyprotein. An artificial -1 frameshift introduced in the natural slippery sequence of the p7-p6 gene junction results in an in-frame GAG-POL-NEF fusion protein due to the absence of ribosomal frameshift. An N-terminal Gly → Ala substitution in gag prevents the formation and release of virus-like particles from transfected cells. This strategy allows for an equimolar production of GAG, POL and NEF proteins and an enhanced MHC Class-I restricted presentation of their CTL epitopes. For safety and regulatory reason, the packaging signal sequence has been removed; the integrase gene deleted; and the reverse transcriptase gene disrupted by insertion of a scrambled *nef* gene at the 3' end of the DNA sequence coding for the RT active site known to be associated with an immunodominant CTL epitope. The *nef* gene has been dislocated by fusing its 5' half to its 3' half without losing its immunodominant CTL epitopes.

### A. NYVAC-HIV-C (vP2010)

### 1. Donor plasmid pMA60gp120C/gagpolnef-C-14.

Donor plasmid pMA60gp120C/GAG-POL-NEF-C-14 was constructed for engineering of NYVAC or MVA expressing HIV-1 clade C gp120 envelope and GAG-POL-NEF proteins. The plasmid is a pUC derivative containing TK left and right flanking sequences in pUC cloning sites. Between two flanking sequences two synthetic early/late (E/L) promoters in a back to back orientation individually drive codon-optimized clade C gp120 gene and *gag-pol-nef* gene. The locations of the TK flanking sequences, E/L promoters, transcriptional termination signal, gp120 and *gag-pol-nef* genes as described in Table II below:

**Table II**

| ***pMA60gp120C*/*gagpolnef-C-14*** | |
|---|---|
| Left flanking sequence | Nt. 1609-2110 (complementary) |
| Right flanking sequence | Nt. 4752-5433 (complementary) |
| | |
| E/L promoter for gp120 | Nt.12-51 |
| Gp120 gene (ATG-TGA) | Nt 61-1557 |
| Terminal signal for gp120 | Nt.1586-1592 |
| | |
| E/L promoter for gagpolnef | Nt. 9794-9833 (complementary) |
| gagpolnef gene (ATG-TAA) | Nt. 5531-9784 (complementary) |
| Terminal signal for gagpolnef | Nt.5422-5416 (complementary) |

### 2. Construction of pMA60gp120C/gagpolnef-C-14 DNA origin:

a. pMA60: This plasmid is a pUC derivative containing TK right and left flanking sequences in pUC cloning sites. Between the two flanking sequences there is a synthetic E/L promoter. The left flanking sequence is located at 37-550 and right flanking sequence is at 610-1329. The E/L promoter (AAAATTGAAATTTTATTTTTTTTTTTTGAAATATAAATA) is located at 680-569.
b. pCR-Script clade C-syngp120: The plasmid contained a codon-optimized clade C HIV-1 gp120 gene. The gp120 gene is located at nucleotides 1-1497 (ATG-TAA).
c. pCRs-cript clade C-syngagpolnef: The plasmid containing a codon-optimized clade C HIV-1 *gagpolnef* gene was provided by Hans Wolf and Walf Wagner (Regensburg University, Germany). The *gagpolnef* gene was located between nucleotides 1-4473 (ATG-TAA).
d. pSE1379.7: The plasmid is a Bluescript derivative containing a synthetic E/L promoter. The E/L promoter is located at nucleotides 1007- 968.

### 3. Construction of pMA60 gp120C/gagpolnef-C-14:

a. Construction of pMA60-T5NT-24: pMA60 has a synthetic E/L promoter but has no transcriptional termination signal for the promoter. To insert a terminal signal T5NT for the promoter, a DNA fragment composed of a pair of oligonucleotides, 5'-CCGGAATTTTTATT-3'(7291) / 3'- TTAAAAATAAGGCC-5' (7292), was inserted into Xma I site on pMA60. The resulted plasmid was designated pMA60-T5NT-24 (notebook 1959, p54, Lisa Murdin, Aventis Canada). A Vector-NTI file for the plasmid was included. In the file the E/L promoter was located at nt.3356-3395 and the T5NT sequence is at nt 3417-3423.
b. Construction of pMA60gp120C-10: To generate a clade C gp120 gene without extra sequence between promoter and start codon ATG a KpnI-KpnI fragment (nt. 4430-1527) containing the gp120 gene was isolated from pCR-Script clade C-syngp120 and used as template in a PCR. In the PCR, primers 7490/ 7491 (7490: 5'-TTGAATTCTCGAGCATGGACAGGGCCAAGCTGCTGCTGCTGCTG and 7491: 5'-TGCTGCTCACGTTCCTGCACTCCAGGGT) were used to amplify a ∼370 bp 5'-gp120 fragment. The fragment was cut with EcoRI and AatII generating an EcoRI-AatII fragment (∼300 bp). The EcoRI-AatII fragment was used to replace a corresponding EcoRI-Aat II fragment (nt. 4432-293) on pCR-Script clade C-syngp120 resulting in a plasmid pCR-Script clade Cgp120-PCR-19. A XhoI-XhoI fragment containing a gp120 gene was isolated from pCR-Script cladeCgp120- PCR-19 and cloned into XhoI site on pMA60-T5NT-24 generating pMA60gp120C-10.
c. Construction of pMA60gp120C/gagpolnef-C-14: To create a clade C gagpolnef gene without extra sequence between promoter and stat codon of the gene a KpnI-KpnI (nt 7313-4352) fragment containing the *gagpolnef* gene was isolated from pCRscript-Syngagpolnef and used as template in a PCR reaction. The primers were oligonucleotides (7618: 5'TTTCTCGAGCATGGCCGCCAGGGCCAGCATCCTGAGG / 7619: 5'-ATCTGCTCCTGCAGGTTGCTGGTGGT). A fragment (∼740 bp) amplified in the PCR was cloned into Sma I site on pUC18 resulting in a plasmid designated pATGgpn-740. The ∼740 bp fragment in pATGgpn-740 was confirmed by DNA sequencing. The pATGgpn-740 was cut with XhoI and StuI generating an XhoI-StuI fragment (∼480 bp). In addition, pCRScript-syngagpolnef was cut with StuI and KpnI generating a StuI-KpnI fragment (nt. 479-4325). Meanwhile pSE1379.7, a Bluescript derivative containing an E/L promoter, was linealized with XhoI and KpnI generating an XhoI-KpnI receptor fragment (- 3 kb). The two fragments (XhoI-Stu I and StuI-KpnI) and the receptor fragment (XhoI-KpnI) were ligated together generating a plasmid pATGgagpolnef-C-2. Finally, the pATG-gagpolnef-C-2 was cut with SalI generating a SalI-SalI fragment that contained an E/L-gagpolnef cassette. The SalI-SalI fragment was cloned into a SalI site on pMA60gp120C-10 generating pMA60gp120C/gagpolnef-C-14.

### 4. Generation of NYVAC-HIV-C recombinant (vP2010)

The IVR was performed by transfection of 1°CEF cells (Merial product) with pMA60gp120C/gagpolenef C-14 using calcium phosphate method and simultaneously infection of the cells with NYVAC as rescue virus at MOI of 10. After ∼14 hr, the transfected-infected cells were harvested, sonicated and used for recombinant virus screening. Recombinant plaques were screened based on plaque lift hybridization method. A 1.5 kb clade C gp120 gene that was labeled with p32 according to a random primer labeling kit protocol (Promega) was used as probe. In the first round screening, ∼11700 plaques were screened and three positive clones designated vP2010-1,vP 2010-2, vP2010-3, were obtained. After sequential four rounds of plaque purification, recombinants designated vP2010-1-2-1-1, vP2010-1-2-2-1, vP2010-1-4-1-1, vP2010-1-4-1-2 and vP2010-1-4-2-1 were generated and confirmed by hybridization as 100% positive using the gp120 probe. P2 stocks of these recombinants were prepared. A P3 (roller bottle) stock with a titer 1.2x 10⁹ was prepared.

### 5. Stability of vP2010

To verify that the NYVAC-HIV-C (vP2010) recombinant could be passaged without lost of transgene expression, a stability test was performed. The recombinant was passaged from P2 stock to P10 in CEF cells with moi of 0.1 and 0.01. Plaques generated in CEF cells with the p10 stocks were analyzed with anti-gp120 monclonal antibody K3A (Virogenetics) and anti-clade C p24 anti serum (Aventis Pasteeur France). The results show that in moi of 0.1, 84 % plaques are positive to gp120 antibody. In moi of 0.01, 76% plaques are positive to gp120 antibody and 100% plaques are positive to p24 antiserum. There was some loss (16-24%) of clade C gp120 expression, even though the virus is relatively stable over 10 passages with a low MOI.

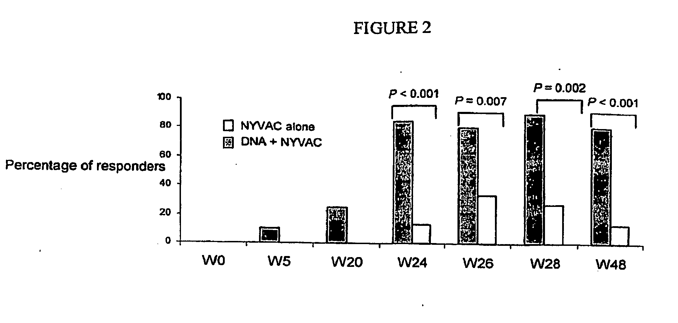

Expression of clade C gp120 and *gagpolnef* from various passaged-vP2010 were verified by Western blot. The CEF cells were infected with various passaged-vP2010 viruses. Cell culture media and cell lysates after the infection were analyzed with anti-gp120 monoclonal antibody K3A (Virogenetics) and anti-p24 serum (Aventis Pasteur in France). Expression of gp120 and gagpolnef from P10 viruses was shown in Fig.1 and Fig.2. The expected gp120 band and GAG-POL-NEF fusion protein band with molecular weight 120-190 kd were observed. Successful expression of gp120 and GAG-POL-NEF from vP201 0 was also demonstrated by immunoplaque assay as mentioned above.

### B. DNA C

The DNA C vector was engineered to contain the components listed above using the pORT system first described by Canenburgh, et al. (Nucleic Acid Res. 2001. 29: e26) (Cobra Biomanufacturing Plc; United Kingdom).

### EXAMPLE 2

### Immunization of Human Beings Against HIV-C

### A. Immunological Compositions

### 1. DNA Vaccine ("DNA C")

DNA C is maintained in liquid form, with an extractable volume of 2 ml to 2.2 ml in 5 ml vials stored at -20°C. The appearance is clear and the composition contains the following components per ml of DNA HIV-C: DNA C (1.05 mg), Tris-HCl (1.57 mg), EDTA (0.372 mg), NaCl (9 mg). These components are brought to one ml with water for injections.

### 2. NYVAC-HIV C (vP2010)

The presentation is in a liquid form, with an extractable volume of 1 ml to 1.1ml in single dose 3ml vials stored at -20°C. The composition contains 10^{7.82} DICC₅₀ NYVAC-HIV C (vP2010), 0.25 ml 10 mM Tris-HCl buffer; pH 7.5, 0.25 ml Virus stabilizer (lactoglutamate); and, 0.5 ml freeze-drying medium.

### B. Clinical Trial Design

The data provided herein reflects the results of a clinical trial in which 40 volunteers were randomised to receive DNA C (naked DNA) or nothing at weeks 0 and 4, followed by NYVAC C at weeks 20 and 24. Administration Regimens 1 (DNA C - NYVAC C prime boost) and 2 (NYVAC C only) are shown in **Table III** below:

**Table III**

| ***Immunization Regimens*** | | | | |
|---|---|---|---|---|
| Regimen | Week 0 | Week 4 | Week 20 | Week 24 |
| 1 Unprimed N=20 | DNA C 2x2ml IM* right and left vastus lateralis | DNA C 2x2ml IM right and left vastus lateralis | NYVAC C IM non-dominant deltoid | NYVACC IM non-dominant deltoid |
| 2 Prime-boost n=20 | Nothing | Nothing | NYVAC C IM non-dominant deltoid | NYVAC C IM non-dominant deltoid |

| | | | | |
|---|---|---|---|---|
| *IM denotes intramuscular administration | | | | |

The main objectives of this trial were to evaluate the safety and immunogenicity of the prime boost regimen (DNA C + NYVAC C) compared to NYVAC C alone. The design was open for participants and clinical investigators, without a placebo control, and 40 volunteers (see description of trial population below) were randomized to receive DNA C or nothing on day 0 and at week 4 followed by NYVAC C at weeks 20 and 24. The participants received two IM injections (right and left vastus lateralis), with each injection containing two ml DNA C in liquid form (1.05 mg per ml and a total dose of 4.2 mg). NYVAC C was administered as a one ml (10^{7.7}CCID₅₀ NYVAC C per ml) in the deltoid. The laboratory investigators undertaking and interpreting the assays were blind to the allocation. The primary endpoints were safety (local and systemic side effects) and immunogenicity. The protocol was determined to be safe and immunogenic, as described below.

The primary immunogenicity endpoints were measured at week 26 and 28 by the quantification of T-cell responses using the IFN-γ ELISPOT assay following a conventional over night stimulation of the blood mononuclear cells with the panel of peptide pools encompassing env, gag, pol and nef of HIV-1 CN54 clade C. The T-cell responses were also measured on day 0 and at weeks 5, 20, 24 and 48. A positive ELISPOT assay was defined as exhibiting > 4-fold more spots than the negative control and > 55 SFU/10⁶ cells (i.e., a "responder"). Individual assays were considered "valid" if the negative control < 50 SFU/10⁶ cells and the positive control (SEB) > 500 SFU/10⁶ cells.

Forty healthy male and female participants in London and Lausanne at low risk of HIV infection were entered into the study. Fifty percent of the enrolled volunteers were females and fifty percent were males. The majority (90%) of volunteers were Caucasians having a median age of 32 years. As a result of preserving the integrity of the randomization, an imbalance between the two groups emerged with 23 participants allocated to receive DNA C and NYVAC C, and 17 allocated to NYVAC C alone. After the first DNA vaccination, two participants were withdrawn from the vaccination scheme due to adverse events, and the second DNA vaccination was given to 21 participants only. The two withdrawn participants did not receive NYVAC C but attended all visits. A further three participants also received no NYVAC: one female received two DNA C immunizations but decided that she did not wish to receive the two NYVAC C immunizations and attended some visits; another two participants were lost to follow-up. The remainder (n=35) received the full vaccination scheme shown in **Table III** and have completed the study (all have reached the 48 week timepoint).

### C. Clinical Trial Results

A significant difference in the proportion of subjects with positive vaccine-induced T-cell responses within the two study groups was observed. The proportion of responders was 90% (18/20) in the DNA C+NYVAC C group compared to 40 % (6/15) in the NYVAC C alone group (*P*=0.003). One of the six responders in the NYVAC C alone group had a very week response just above background (in the range of 60 SFU/10⁶ cells) at weeks 26 and 28 but also at weeks 0, 5 and 20 prior to vaccination. Although due to the study design, this subject had to be considered positive at weeks 26 and 28, the T-cell response observed was clearly non-specific and for these reasons it was not further considered in the additional analyses. It was thereby concluded that the proportion of subjects with vaccine-induced specific T-cell responses was 33% (5 out of 15) in the group vaccinated with NYVAC C alone. The proportion of responders after the DNA C vaccination was very low after two vaccinations (2/18 or 12.5% at week 5, 4/18 or ∼22% at week 20) (**Fig. 2**). Furthermore, the proportion of responders in the DNA C + NYVAC C group mostly peaked (17 out of 20) after the first NYVAC C boost and the proportion of responders was still 75% at week 48, i.e. 6 months after the completion of the vaccination. Only two subjects within the NYVAC C alone group had still positive vaccine-induced T-cell responses at week 48.

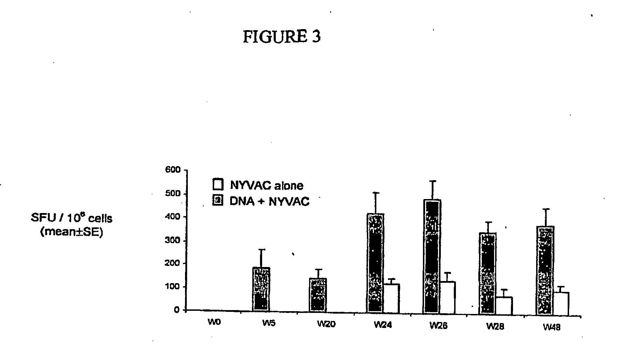

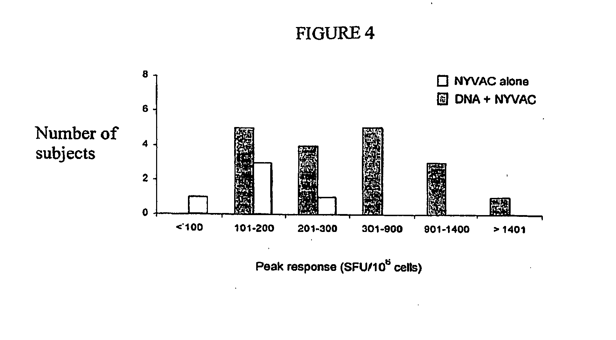

Vaccine-induced T-cell responses were also assessed using the IFN-γ ELISPOT assay following stimulation of blood mononuclear cells with a panel of 464 peptides (15-mers overlapping by 11 amino acids) grouped in 8 pools (50-60 peptides per pool). The peptides encompassed the env, gag, pol and nef proteins of HIV-1 and were designed based on the sequence of the immunogens expressed by the DNA and NYVAC that were derived from the CN54 clade C isolate. Vaccine-induced T-cell responses were predominantly directed against env in both DNA C + NYVAC C and NYVAC C alone groups. Env-specific responses were observed in 22 out of 23 responders in both groups while gag, pol and nef vaccine-induced T-cell responses were only observed in 20% of volunteers (data not shown). The responses against gag, pol and nef were generally transient and substantially lower in magnitude compared to the env-specific responses. The env-specific T-cell responses following DNA C + NYVAC C vaccination were significantly greater compared to the NYVAC alone group. At the time of peak response (week 26), the mean measurement of IFN-γ secreting T-cells was 450 SFU/10⁶ cells in the DNA C + NYVAC C group and 110 SFU/10⁶ cells within NYVAC C alone group (**Fig. 3**). The differences in the magnitude of T-cell response between the two groups were significant (*P*=0.016). Consistent with the substantial difference in the magnitude of the T-cell response between the two groups, the 5-responders within the NYVAC C alone group had most (4 out of 5) of the T-cell response below 200 SFU/10⁶ cells while nine of the 18 responders within the DNA C+NYVAC C group had T-cell responses greater than 300 SFU/10⁶ cells (**Fig. 4**).

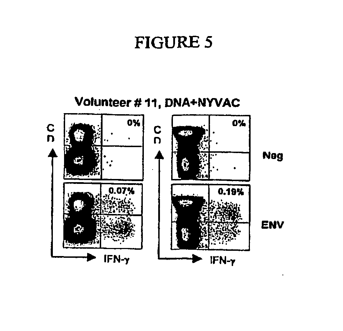

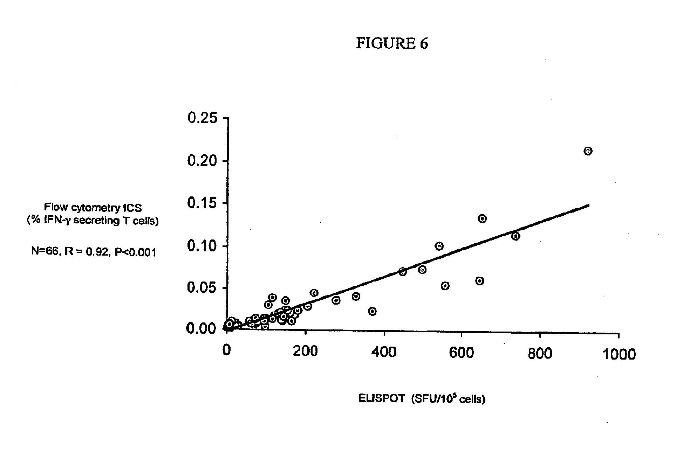

The distribution of vaccine-induced T-cell responses in CD4 and CD8 T-cell populations was assessed in three of the five responders in the NYVAC C alone group and in 16 of 18 responders in the DNA C + NYVAC C group. Only responders with more than 100 SFU/10⁶ blood mononuclear cells measured in the IFN-γ ELISPOT assay were characterised using polychromatic flow cytometry. The vaccine-induced T-cell responses were mediated by CD4 T-cells in all the investigated 19 responders (three in the NYVAC alone and 16 in the DNA C + NYVAC C groups). Vaccine-induced CD8 T-cell responses were additionally observed one of the three responders in the NYVAC C alone group and in seven of 16 responders in the DNA C + NYVAC C groups. Representative flow cytometry profiles of env-specific IFN-γ secreting CD4 and CD8 T-cell responses in responder #11 vaccinated with DNA C+NYVAC C are shown in **Fig. 5**. The characterization of vaccine-induced CD4 and CD8 T-cell responses was performed mostly for env-specific responses since the frequency and the magnitude of the T-cell responses observed against gag, pol and nef was very low and generally below 100 SFU/10⁶ cells. Of note, the polychromatic flow cytometry analysis allowed us to provide an independent confirmation of the responses assessed using the IFN-γ ELISPOT assay. The frequencies of IFN-γ secreting T-cells measured by both assays were compared in 19 responders. It is important to underscore that there was a very high correlation between the frequencies of IFN-γ secreting T-cells measured by the ELISPOT assay and flow cytometry (**Fig. 6**).

The panel of T-cell functions analyzed included IL-2, TNF-α and IFN-γ secretion and proliferation for both CD4 and CD8 T-cells and also degranulation activity for CD8 T-cells. Env-specific CD4 and CD8 T-cells functions were analysed using polychromatic flow cytometry. T-cell functions were analysed after stimulation with env peptide pools. For example, responder #11 (vaccinated with DNA C + NYVAC C) had both env-specific CD4 and CD8 T-cell responses. On the basis of the analysis of IL-2 and IFN-γ secretion, three distinct env-specific CD4 T-cell populations were identified: a) single IL-2, b) dual IL-2/IFN-γ and single IFN-γ. The three functionally distinct populations of env-specific CD4 T-cells were equally represented. Env-specific CD4 T-cells were also able to secrete TNF-α and we identified two populations, i.e. single TNF-α and dual TNF-α/IFN-γ secreting CD4 T-cell populations which were equally represented. Furthermore, vaccine-induced CD4 T-cells efficiently proliferated after stimulation with the env peptide pools.

Similar to CD4 T-cells, the analysis of IL-2 and IFN-γ secretion in CD8 T-cells identified two distinct env-specific CD8 T-cell populations: a) dual IL-2/IFN-γ and single IFN-γ secreting cell populations. It was found that the majority (70%) of env-specific CD8 T-cells were single IFN-γ secreting cells and the remaining cells were dual IL-2/IFN-γ. Almost the totality of IFN-γ secreting CD8 T-cells were also able to secrete TNF-α and were therefore dual TNF-α/IFN-γ secreting cells. A substantial proportion of env-specific CD8 T-cells had degranulation activity following antigen-specific stimulation as indicated by the expression of CD107. Finally, vaccine-induced CD8 T-cells were endowed with proliferation capacity following env-specific stimulation. Similar functional profiles of vaccine-induced CD4 and CD8 T-cell responses were confirmed in six additional vaccinees. Taken together, these results indicated that vaccination with DNA C + NYVAC C induced polyfunctional env-specific CD4 and CD8 T-cell responses.

Phenotypic analysis of vaccine-induced T-cell responses was performed in volunteer #26 vaccinated with DNA C + NYVAC C. Both env-specific CD4 and CD8 T-cells were induced following vaccination. Blood mononuclear cells of volunteer #26 were collected at different time points (week 24, 26 and 48) and were stimulated with env derived peptide pools for 16 hours and stained with CD4, CD8, CD45RA, CCR7, IL-2 and IFN-γ antibodies. It has been previously demonstrated that CD45RA and CCR7 define functionally distinct populations of memory antigen-specific CD4 and CD8 T-cells. The totality (single IL-2+dual IL-2/IFN-γ+single IFN-γ) of env-specific CD4 T-cells were CD45RA-CCR7- and the phenotypic profile and percentage of env-specific CD4 T-cells remained unchanged over time.

In volunteer #26, Env-specific CD8 T-cells (dual IL-2/IFN-γ+ single IFN-γ) were almost equally distributed within CD45RA-CCR7- and CD45RA+CCR7- cell populations at week 24. However; there was a progressive loss of the CD45RA-CCR7- env-specific CD8 T-cell population over time and about 90% of the vaccine-induced CD8 T-cells were CD45RA+CCR7- at week 48. The changes in phenotype and in the percentage of env-specific CD8 T-cells were observed only for vaccine-induced CD8 T-cells since the phenotype and the percentage of EBV/CMV-specific CD8 T-cell responses assessed in blood samples collected at the same time points in volunteer #26 remained unchanged. Similar results were obtained in three additional volunteers.

Identification of peptides/epitopes recognized by vaccine-induced CD4 and CD8 T-cell populations was performed in nine volunteers, eight belonging to the DNA C + NYVAC C and one to the NYVAC C alone groups. Peptides/epitopes characterization was limited to the env-specific responses. After the initial screening using env derived peptide pools, identification of the peptides/epitopes recognized was performed by testing the reactivity of blood mononuclear cells against the relevant peptides in a matrix setting using the IFN-γ ELISPOT assay. Following this analysis, 19 different peptides/epitopes were identified in the nine volunteers studied and further characterization of the vaccine-induced CD4 and CD8 T-cell populations recognizing these peptides/epitopes was performed using polychromatic flow cytometry (**Table 4**).

**TABLE 4**

| Type of Antigen | Peptide sequence | HIV Region |
|---|---|---|
| Class II | VGNLWVTVYYGVPVW | C1/C2 |
| | WVTVYYGVPVWKGAT | C1/C2 |
| | GATTTLFCASDAKAY | C1/C2 |
| | TTLFCASDAKAYDTE | C1/C2 |
| | THACVPADPNPQEMV | C1/C2 |
| | ENVTENFNMWKNEMV | C1/C2 |
| | ENFNMWKNEMVNQMQ | C1/C2 |
| | EMVNQMQEDVISLWD | C1/C2 |
| | CVKLTPLCVTLECRN | C1/C2 |
| | NCSFNATTVVRDRKQ | V1/V2 |
| | NATTVVRDRKQTVYA | V1/V2 |
| | VYALFYRLDIVPLTK | C3 |
| | FYRLDIVPLTKKNYS | C3 |
| | INCNTSAITQACPKV | C3 |
| | PKVTFDPIPIHiYCTP | C3 |
| | FDPIPIHYCTPAGYA | C3 |
| | TGDIIGDIRQAHCNI | V3/C4 |
| | SSSIITIPCRIKQII | V4/V5 |
| | ITIPCRIKQITNMWQ | C5 |
| | CRIKQIINMWQEVGR | C5 |
| | VGRAMYAPPIKGNIT | C5 |
| | MYAPPIKGNITCKSN | C5 |
| | PIKGNITCKSNITGL | C5 |
| | ETFRPGGGDMRNNWR | C5 |
| | ELYKYKVVEIKPLGV | C5 |
| | YKVVEIKPLGVAPTT | C5 |
| | EIKPLGVAPTTTKRR | C5 |
| Class I | LGVAPTTTKRRVVER | C5 |
| HLA-A*01 | YSENSSEYY | V1/V2 |

A variable number of peptide/epitopes, ranging from two to eight, were recognized in each volunteer with a mean of 4.2 peptides / epitope. Ten out of 19 peptides/epitopes identified in the nine volunteers have similarities to those previously identified in subjects with chronic HIV-1 infection or in vaccine studies performed in mice and humans.

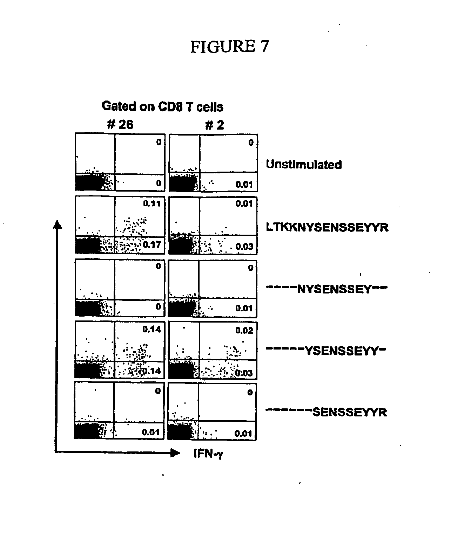

Representative flow cytometry profiles of vaccine-induced env-specific CD4 and CD8 T-cells recognizing individual peptides/epitopes are shown in **Fig. 7**. Fine epitope mapping of the peptide LTKKNYSENSSEYYR recognized by CD8 T-cells in seven volunteers (six belonging to the DNA C + NYVAC C and one to the NYVAC C alone groups) was performed. Using a set of overlapping peptides, it was determined that the epitope recognized by vaccine-induced CD8 T-cells corresponded to the sequence YSENSSEYY (two representative examples).

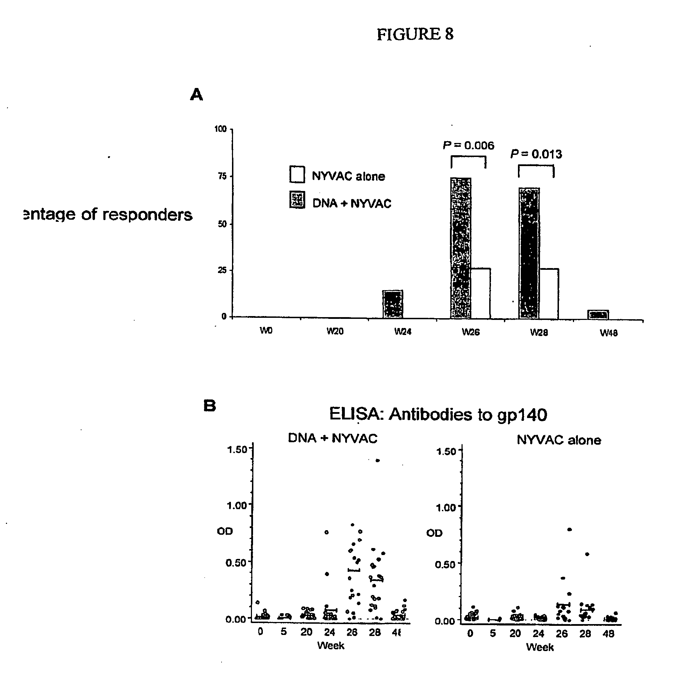

Vaccine-induced IgG antibodies against gp140 CN54 were assessed at different time points during the vaccination regimen (**Figs. 8A and 8B**). The induction of IgG anti-gp140 CN54 was assessed in an ELISA assay. Only a small number of volunteers (25%) had a measurable antibody response at week 26, i.e. 2 weeks after the second NYVAC C immunization, in the NYVAC C alone group. No responders were present at week 48. A large percentage (75%) of volunteers had measurable IgG anti-gp140 antibodies at week 26 in the DNA C+NYVAC C group. No antibody response was detected after the DNA immunization and only 10% of volunteers responded after the first NYVAC C immunization. However, similar to the NYVAC C alone group, the vaccine-induced antibody response was transient and only 5% of volunteers had measurable antibody response at week 48. In addition to the significant differences in the percentage of responders between the two study groups, the magnitude of the antibody response was also significantly greater in the DNA C + NYVAC C group compared to the NYVAC C alone group.

The neutralization activity of the vaccine-induced antibodies was assessed in three different assays including a) a multiple rounds neutralization assay on blood mononuclear cells using the homologous primary isolate CN54, b) a neutrlization assay in a single cycle infection of primary isolate Bx08 in the engineered cell line TZMbI, and c) a neutralization assay using Bal replication in macrophages. The vaccine-induced antibodies failed to show any neutralizing activity.

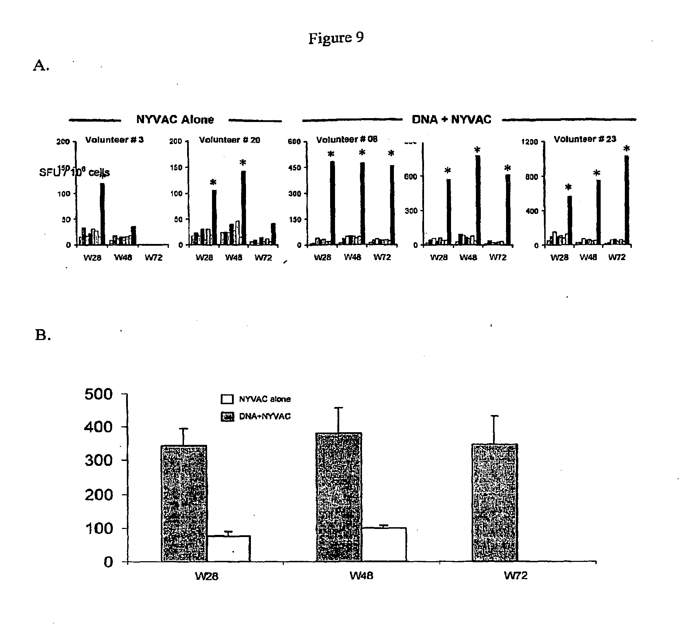

The duration of the study in the original protocol was 48 weeks. However, in order to have insights on the long-term durability of the vaccine-induced T-cell response, the protocol was subsequently amended to assess the T-cell responses at week 72, i.e. one year after the last immunization. The protocol was amended only in Lausanne and, after IRB approval, blood was collected at week 72 only in those volunteers that were originally enrolled in Lausanne and had a positive IFN-γ ELISPOT assay at week 48. Thirteen volunteers (11 belonging to the DNA C+NYVAC C group and 2 to the NYVAC C alone group) were analyzed at week 72 (**Fig. 9A and 9B**). None of the two volunteers belonging to the NYVAC C alone group had a positive IFN-γ T-cell response at week 72. Nine out of the 11 volunteers belonging to the DNA C+NYVAC C group had a positive IFN-γ T-cell response at week 72. Of interest, the magnitude of the IFN-γ T-cell response observed at week 72 was unchanged compared to that measured in the 9 volunteers at week 28 and 48.

While the present invention has been described in terms of the preferred embodiments, it is understood that variations and modifications will occur to those skilled in the art. Therefore, it is intended that the appended claims cover all such equivalent variations that come within the scope of the invention as claimed.

## Claims

1. A two-part immunological composition for use in the treatment or prevention of human immunodeficiency virus (HIV) infection comprising a first form of an HIV immunogen and a second form of an HIV immunogen, the first forms being a DNA molecule and the second form being a NYVAC viral vector, each form encoding HIV immunogens comprising gp120, gag, pol and nef, wherein the forms are administered separately such that administration of the first form enhances an immune response against the subsequently administered second form relative to the administration of the second form alone, wherein the immune response comprises a dominant CD4 T cell response.

2. The composition for use of claim 1 wherein the HIV immunogen is encoded by a gagpolnef cassette of plasmid pMA60gp120C/gagpolnef-C-14 illustrated in Figure 1.

3. The composition for use of claims or 2 wherein the HIV immunogen is encoded by the gagpolnef cassette of Figure 1.

4. The composition for use of any one of claims 1-3 wherein the immunogen is derived from HIV-1 intersubtype (C/B').

5. The composition for use of any one of claims 1-4 wherein the cellular response comprises polyfunctional CD4 T cells.

6. The composition for use of any one of claims 1-5 wherein the cellular response demonstrates at least two characteristics selected from the group consisting of:
a. a high proportion of immunogen-specific CD4 cells within the population of total responding T cells;
b. responding CD4 T cells that form up to about 1,000 or more spot-forming units (SFUs) by ELISPOT assay per one million blood mononuclear cells;
c. polyfunctional CD4 T cells;
d. responding CD4 T cells secret both IL-2 and IFN-γ; and
e. the dominant CD4 T cell immune response encompasses at least two epitopes of an HIV immunogen.

7. The composition for use of any one of claims 1-6 wherein the cellular response further comprises CD8 cytotoxic T cells.

8. A composition for use of any one of claims 1-7 utilized in conjunction with at least one antiviral agent selected from the group consisting of an anti-HIV agent, a protease inhibitor, an HIV entry inhibitor, a reverse transcriptase inhibitor, and an anti- retroviral nucleoside analog, and wherein the at least one antiviral agent may be selected from the group consisting of Agenerase (amprenavir), Combivir (Retrovir, Epivir), Crixivan (indinavin), Emtriva (emtricitabine), Epivir (3TC, lamivudine), Epzicom, Fortovase (Invirase, saquinavir), Fuzeon(enruvirtide), Hivid (DDC, zalcitabine), Kaletra (lopinavir), Lexiva, (Fosamprenavir), Norvir (ritonavir), Rescriptor (delavirdine), Retrovir (AZT, zidovudine), Reyatax (atazarsavir, BMS-232632), Sustiva (efavirenz), Trizivir (abacavir, zidovudine, lamivudine), Truvada (Emtricitabine / Tenofovir DF), Videx (ddl, didanosine), Videx EC (didanosine) Viracept (nevirapine), Viread (tenofovir disoproxil fumarate), Zerit, (d4T, stavudine), and Ziagen (abacavir).

## Patentansprüche

1. Eine zweiteilige immunologische Zusammensetzung zur Verwendung bei der Behandlung oder Prävention einer Infektion mit dem humanen Immundefizienzvirus (HIV), welche eine erste Form eines HIV-Immunogens und eine zweite Form eines HIV-Immunogens umfasst, wobei die erste Form ein DNA-Molekül ist und die zweite Form ein viraler NYVAC-Vektor ist, wobei jede Form HIV-Immunogene codiert, die gp120, gag, pol und nef umfassen, wobei die Formen getrennt verabreicht werden, so dass die Verabreichung der ersten Form eine Immunantwort auf die anschließend verabreichte zweite Form in Bezug auf die Verabreichung der zweiten Form allein verstärkt, wobei die Immunantwort eine dominante CD4-T-Zellantwort umfasst.

2. Die Zusammensetzung zur Verwendung nach Anspruch 1, wobei das HIV-Immunogen von einer gagpolnef-Kassette des Plasmids pMA60gp120C/gagpolnef-C-14, das in Figur 1 dargestellt ist, codiert wird.

3. Die Zusammensetzung zur Verwendung nach den Ansprüchen 1 oder 2, wobei das HIV-Immunogen von der gagpolnef-Kassette aus Figur 1 codiert wird.

4. Die Zusammensetzung zur Verwendung nach irgendeinem der Ansprüche 1 - 3, wobei das Immunogen vom HIV-I Intersubtyp (C/B') abgeleitet ist.

5. Die Zusammensetzung zur Verwendung nach irgendeinem der Ansprüche 1 - 4, wobei die Zellantwort polyfunktionale CD4-T-Zellen umfasst.

6. Die Zusammensetzung zur Verwendung nach irgendeinem der Ansprüche 1 - 5, wobei die Zellantwort wenigstens zwei charakteristische Merkmale zeigt, die ausgewählt sind aus der Gruppe, bestehend aus:
a. einem hohen Anteil an Immunogen-spezifischen CD4-Zellen innerhalb der Population der gesamten antwortenden T-Zellen;
b. antwortenden CD4-T-Zellen, die bei einem ELISPOT-Test pro einer Million mononukleärer Blutzellen bis zu ca. 1.000 oder mehr einen Fleck (spot) bildende Einheiten (SFUs) bilden;
c. polyfunktionalen CD4-T-Zellen;
d. antwortenden CD4-T-Zellen, die sowohl IL-2 als auch IFN-γ sezernieren; und
e. der dominanten CD4-T-Zell-Immunantwort, die wenigstens zwei Epitope eines HIV-Immunogens umfasst.

7. Die Zusammensetzung zur Verwendung nach irgendeinem der Ansprüche 1 - 6, wobei die Zellantwort weiterhin zytotoxische CD8-T-Zellen umfasst.

8. Eine Zusammensetzung zur Verwendung nach irgendeinem der Ansprüche 1 - 7, welche in Verbindung mit wenigstens einem antiviralen Mittel benutzt wird, das ausgewählt ist aus der Gruppe, bestehend aus einem anti-HIV-Mittel, einem Proteaseinhibitor, einem HIV-Eintrittsinhibitor, einem Inhibitor der reversen Transkriptase und einem anti-retroviralen Nukleosid-Analogon, und wobei das wenigstens eine antivirale Mittel ausgewählt werden kann aus der Gruppe, bestehend aus Agenerase (Amprenavir), Combivir (Retrovir, Epivir), Crixivan (Indinavir), Emtriva (Emtricitabin), Epivir (3TC, Lamivudin), Epzicom, Fortovase (Invirase, Saquinavir), Fuzeon (Enfuvirtid), Hivid (DDC, Zalcitabin), Kaletra (Lopinavir), Lexiva (Fosamprenavir), Norvir (Ritonavir), Rescriptor (Delavirdin), Retrovir (AZT, Zidovudin), Reyatax (Atazanavir, BMS-232632), Sustiva (Efavirenz), Trizivir (Abacavir, Zidovudin, Lamivudin), Truvada (Emtricitabin / Tenofovir DF), Videx (ddl, Didanosin), Videx EC (Didanosin), Viracept (Nevirapin), Viread (Tenofovir Disoproxil Fumarat), Zerit (d4T, Stavudin) und Ziagen (Abacavir).

## Revendications

1. Composition immunologique en deux parties destinée à être utilisée dans le traitement ou la prévention d'une infection par le virus de l'immunodéficience humaine (VIH), comprenant une première forme d'un immunogène du VIH et une seconde forme d'un immunogène du VIH, la première forme étant une molécule d'ADN et la seconde forme étant un vecteur viral NYVAC, chaque forme codant pour des immunogènes du VIH comprenant gp120, gag, pol et nef, dans laquelle les formes sont administrées séparément de sorte que l'administration de la première forme stimule une réponse immunitaire contre la seconde forme administrée ultérieurement par rapport à l'administration de la seconde forme seule, dans laquelle la réponse immunitaire comprend une réponse dominante de cellules T CD4.

2. Composition selon la revendication 1 destinée à être utilisée, dans laquelle l'immunogène du VIH est codé par une cassette gagpolnef du plasmide pMA60gp120C/gagpolnef-C-14 illustré par la Figure 1.

3. Composition selon les revendications 1 ou 2 destinée à être utilisée, dans laquelle l'immunogène du VIH est codé par la cassette gagpolnef de la Figure 1.

4. Composition selon l'une quelconque des revendications 1 à 3 destinée à être utilisée, dans laquelle l'immunogène est dérivé du VIH-1 inter sous-type (C/B').

5. Composition selon l'une quelconque des revendications 1 à 4 destinée à être utilisée, dans laquelle la réponse cellulaire comprend des cellules T CD4 polyfonctionnelles.

6. Composition selon l'une quelconque des revendications 1 à 5 destinée à être utilisée, dans laquelle la réponse cellulaire démontre au moins deux caractéristiques sélectionnées dans le groupe consistant en :
a. une forte proportion de cellules CD4 spécifiques à un immunogène au sein de la population de cellules T répondeuses totales ;
b. des cellules T CD4 répondeuses qui forment jusqu'à environ 1 000 unités formant des spots (SFU) ou davantage par un test ELISPOT pour un million de cellules mononucléaires sanguines ;
c. des cellules T CD4 polyfonctionnelles ;
d. des cellules T CD4 répondeuses qui sécrètent à la fois l'IL-2 et l'IFN-γ ; et
e. la réponse immunitaire dominante de cellules T CD4 englobe au moins deux épitopes d'un immunogène du VIH.

7. Composition selon l'une quelconque des revendications 1 à 6 destinée à être utilisée, dans laquelle la réponse cellulaire comprend en outre des cellules T cytotoxiques CD8.

8. Composition selon l'une quelconque des revendications 1 à 7 destinée à être utilisée, où la composition est utilisée en association avec au moins un agent antiviral sélectionné dans le groupe consistant en un agent anti-VIH, un inhibiteur de protéase, un inhibiteur d'entrée du VIH, un inhibiteur de la transcriptase inverse, et un analogue nucléosidique antirétroviral, et dans laquelle le au moins un agent antiviral peut être sélectionné dans le groupe consistant en l'Agenerase (amprénavir), le Combivir (Retrovir, Epivir), le Crixivan (indinavir), l'Emtriva (emtricitabine), l'Epivir (3TC, lamivudine), l'Epzicom, le Fortovase (Invirase, saquinavir), le Fuzeon (enfuvirtide), l'Hivid (DDC, zalcitabine), le Kaletra (lopinavir), le Lexiva (Fosamprénavir), le Norvir (ritonavir), le Rescriptor (delavirdine), le Retrovir (AZT, zidovudine), le Reyatax (atazanavir, BMS-232632), le Sustiva (éfavirenz), le Trizivir (abacavir, zidovudine, lamivudine), le Truvada (Emtricitabine/Ténofovir DF), le Videx (ddI, didanosine), le Videx EC (didanosine), le Viracept (névirapine), le Viread (fumarate de ténofovir disoproxil), le Zerit (d4T, stavudine), et le Ziagen (abacavir).
